# EUROPEAN PATENT APPLICATION

(11) **EP 4 792 849 A2**
(43) Date of publication of application: **19.08.2026**
(21) Application number: 26159451.9
(22) Date of filing: 18.02.2026
(51) Int. Cl.: A61M 1/34, A61M 1/36

(54) **PANEL KIT FOR BLOOD PURIFICATION DEVICE**

(30) Priority: 18.02.2025 CN 202510180720; 04.02.2026 CN 202610166296
(71) Applicant: Shenzhen Mindray Bio-Medical Electronics Co., Ltd., Shenzhen, Guangdong 518057 (CN)
(72) Inventor: ZOU, Zhihua, Shenzhen, 518057 (CN); LUO, Caijin, Shenzhen, 518057 (CN); WEI, Kai, Shenzhen, 518057 (CN); ZOU, Huan, Shenzhen, 518057 (CN); AI, Shiming, Shenzhen, 518057 (CN)
(74) Representative: KIPA AB

(57) **Abstract**

Disclosed is a panel kit for a blood purification device, including a power component and a monitoring component, the power component includes a power panel configured to be detachably mounted to a host and at least two pump pipeline sections mounted to the power panel, and if the power panel is mounted at a first target position of the host, the pump pipeline sections cooperate with pump components respectively to drive a blood and a medical liquid to flow, the monitoring component includes a monitoring panel can be detachably mounted to the host and at least two monitoring pipeline sections mounted to the monitoring panel; at least one of the monitoring pipeline sections is provided with a second interface; the monitoring component can be manually mounted at a second target position of the host and a second interface is synchronously connected to a first interface of the host.

## Description

### CROSS-REFERENCE TO RELATED APPLICATION

This application claims priority to Chinese patent application No. 202510180720.9 filed at China National Intellectual Property Administration (CNIPA) on February 18, 2025 and entitled "PANEL KIT FOR BLOOD PURIFICATION DEVICE" and Chinese patent application No. 202610166296.7 filed at China National Intellectual Property Administration (CNIPA) on February 4, 2026 and entitled "PANEL KIT FOR BLOOD PURIFICATION DEVICE".

### TECHNICAL FIELD

The application belongs to the field of medical devices, and particularly relates to a panel kit for a blood purification device.

### BACKGROUND

Blood purification therapy is a treatment method that draws a patient's blood out of the patient's body and then removes pathogenic substances from the patient's blood by a filter element, to achieve purification of the blood; main treatment methods include hemodialysis, hemofiltration, hemodiafiltration, hemoperfusion, plasma exchange, etc.

In the related art, the blood purification therapy is usually achieved by connecting a blood purification device to the patient's blood vessel. Therefore, it needs to provide many pipeline sections or the like on the blood purification device, to deliver the patient's blood, replacement fluid, anticoagulant, etc. Furthermore, in order to ensure safety of the blood flowing back to the patient's body, the pipeline sections should be replaced after each time they are used, then there is a large difficulty of mounting the pipeline sections since there is a large number of pipeline sections.

### SUMMARY

According to a first aspect, an embodiment of the application provides a panel kit for a blood purification device, the blood purification device includes a host, the host is provided with pump components and a first interface, and the panel kit includes a power component and a monitoring component.

The power component includes a power panel and at least two pump pipeline sections, the power panel is configured to be detachably mounted to the host, the at least two pump pipeline sections are mounted to the power panel, at least one of the pump pipeline sections is configured to cooperate with at least a corresponding one of the pump components so as to drive a blood to flow along the at least one of the pump pipeline sections, and at least one of the pump pipeline sections is configured to cooperate with at least corresponding one of the pump components so as to drive a medical liquid to flow along the at least one of the pump pipeline sections; if the power panel is mounted at a first target position of the host, the pump pipeline sections are adapted to the pump components respectively, and if the pump pipeline sections are adapted to the pump components respectively, the pump pipeline sections are capable of being driven by the pump components respectively to drive the corresponding blood or medical liquid to flow.

The monitoring component includes a monitoring panel and at least two monitoring pipeline sections, the monitoring panel is configured to be detachably mounted to the host, the monitoring panel is arranged separately from the power panel such that the monitoring panel and the power panel are independent of each other and can be assembled and disassembled separately, the at least two monitoring pipeline sections are mounted to the monitoring panel, the at least two monitoring pipeline sections are configured to monitor parameter information of the medical liquid and/or the blood, and the parameter information at least includes a pressure parameter; at least one of the monitoring pipeline sections is provided with a second interface; the monitoring component is capable of being manually mounted such that the monitoring panel is mounted at a second target position of the host and the second interface is synchronously connected to the first interface, and if the second interface is connected to the first interface, the monitoring pipeline sections are used for the host to monitor the parameter information.

According to a second aspect, an embodiment of the application further provides a panel kit for a blood purification device, the blood purification device includes a host, the host is provided with pump components and a retractable first interface, and the panel kit includes a power component and a monitoring component.

The power component includes a power panel and at least two pump pipeline sections, the power panel is configured to be detachably mounted to the host, the at least two pump pipeline sections are mounted to the power panel, at least one of the pump pipeline sections is configured to cooperate with at least a corresponding one of the pump components so as to drive a blood to flow along the at least one of the pump pipeline sections, and at least one of the pump pipeline sections is configured to cooperate with at least corresponding one of the pump components so as to drive a medical liquid to flow along the at least one of the pump pipeline sections; if the power panel is mounted at a first target position of the host, the pump pipeline sections are adapted to the pump components respectively, and if the pump pipeline sections are adapted to the pump components respectively, the pump pipeline sections are capable of being driven by the pump components respectively to drive the corresponding blood or medical liquid to flow.

The monitoring component includes a monitoring panel and at least two monitoring pipeline sections, the monitoring panel is configured to be detachably mounted to the host, the monitoring panel is arranged separately from the power panel such that the monitoring panel and the power panel are independent of each other and can be assembled and disassembled separately, the at least two monitoring pipeline sections are mounted to the monitoring panel, the at least two monitoring pipeline sections are configured to monitor parameter information of the medical liquid and/or the blood, and the parameter information at least includes a pressure parameter; at least one of the monitoring pipeline sections is provided with a second interface, and if the monitoring panel is mounted at a second target position of the host, the first interface can be extended to be connected to the second interface, and if the second interface is connected to the first interface, the monitoring pipeline sections are used for the host to monitor the parameter information.

According to a third aspect, an embodiment of the application further provides a panel kit for a blood purification device, the blood purification device includes a host, the host is provided with pump components and a first interface, and the panel kit includes a power component and a monitoring component.

The power component includes a power panel and at least two pump pipeline sections, the power panel is configured to be detachably mounted to the host, the at least two pump pipeline sections are mounted to the power panel, at least one of the pump pipeline sections is configured to cooperate with at least a corresponding one of the pump components so as to drive a blood to flow along the at least one of the pump pipeline sections, and at least one of the pump pipeline sections is configured to cooperate with at least corresponding one of the pump components so as to drive a medical liquid to flow along the at least one of the pump pipeline sections; if the power panel is mounted at a first target position of the host, the pump pipeline sections are adapted to the pump components respectively, and if the pump pipeline sections are adapted to the pump components respectively, the pump pipeline sections are capable of being driven by the pump components respectively to drive the corresponding blood or medical liquid to flow.

The monitoring component includes a monitoring panel and at least two monitoring pipeline sections, the monitoring panel is configured to be detachably mounted to the host, the monitoring panel is arranged separately from the power panel such that the monitoring panel and the power panel are independent of each other and can be assembled and disassembled separately, the at least two monitoring pipeline sections are mounted to the monitoring panel, the at least two monitoring pipeline sections are configured to monitor parameter information of the medical liquid and/or the blood, and the parameter information at least includes a pressure parameter; at least one of the monitoring pipeline sections is provided with a second interface; if the monitoring panel is mounted at a second preset position of the host, the host is capable of driving the monitoring panel to move to a second target position, such that the second interface is connected to the first interface, and if the second interface is connected to the first interface, the monitoring pipeline sections are used for the host to monitor the parameter information.

According to a fourth aspect, an embodiment of the application further provides a panel kit for a blood purification device, the blood purification device includes a host, the host is provided with pump components and a first interface, and the panel kit includes a power component and a monitoring component.

The power component includes a power panel and at least two pump pipeline sections, the power panel is configured to be detachably mounted to the host, the at least two pump pipeline sections are mounted to the power panel, at least one of the pump pipeline sections is configured to cooperate with at least a corresponding one of the pump components so as to drive a blood to flow along the at least one of the pump pipeline sections, and at least one of the pump pipeline sections is configured to cooperate with at least corresponding one of the pump components so as to drive a medical liquid to flow along the at least one of the pump pipeline sections; if the power panel is mounted at a first target position of the host, the pump pipeline sections are adapted to the pump components respectively, and if the pump pipeline sections are adapted to the pump components respectively, the pump pipeline sections are capable of being driven by the pump components respectively to drive the corresponding blood or medical liquid to flow.

The monitoring component includes a monitoring panel and at least two monitoring pipeline sections, the monitoring panel is configured to be detachably mounted to the host, the monitoring panel is arranged separately from the power panel such that the monitoring panel and the power panel are independent of each other and can be assembled and disassembled separately, the at least two monitoring pipeline sections are mounted to the monitoring panel, the at least two monitoring pipeline sections are configured to monitor parameter information of the medical liquid and/or the blood, and the parameter information at least includes a pressure parameter; at least one of the monitoring pipeline sections is provided with a second interface; the second interface is movably arranged relative to the monitoring panel, and if the monitoring panel is mounted at a second target position of the host, the second interface is capable of being manually operated to achieve connection of the first interface to the second interface, and if the second interface is connected to the first interface, the monitoring pipeline sections are used for the host to monitor the parameter information.

According to a fifth aspect, an embodiment of the application further provides a panel kit for a blood purification device, the blood purification device includes a host, the host is provided with pump components, a conductive member and a first interface, and the panel kit includes a power component and a monitoring component.

The power component includes a power panel and at least two pump pipeline sections, the power panel is configured to be detachably mounted to the host, the at least two pump pipeline sections are mounted to the power panel, at least one of the pump pipeline sections is configured to cooperate with at least a corresponding one of the pump components so as to drive a blood to flow along the at least one of the pump pipeline sections, and at least one of the pump pipeline sections is configured to cooperate with at least a corresponding one of the pump components so as to drive a medical liquid to flow along the at least one of the pump pipeline sections; if the power panel is mounted at a first target position of the host, the pump pipeline sections are adapted to the pump components respectively, and if the pump pipeline sections are adapted to the pump components respectively, the pump pipeline sections are capable of being driven by the pump components respectively to drive the corresponding blood or medical liquid to flow.

The monitoring component includes a monitoring panel, monitoring pipeline sections and an electrostatic discharge (ESD) element, the monitoring panel is configured to be detachably mounted to the host, the monitoring panel is arranged separately from the power panel such that the monitoring panel and the power panel are independent of each other and can be assembled and disassembled separately, each of the monitoring pipeline sections and the ESD element are mounted to the monitoring panel, the monitoring pipeline sections are configured to monitor parameter information of the medical liquid and/or the blood, and the parameter information at least includes a pressure parameter; at least one of the monitoring pipeline sections is provided with a second interface; the monitoring component is capable of being manually mounted such that the monitoring panel is mounted at a second target position of the host, the second interface is synchronously connected to the first interface and the ESD element is synchronously connected to the conductive member, and if the second interface is connected to the first interface, the monitoring pipeline sections are used for the host to monitor the parameter information, and if the ESD element is connected to the conductive member, the ESD element is capable of discharging static electricity from the panel kit.

According to a sixth aspect, an embodiment of the application further provides a panel kit for a blood purification device, the blood purification device includes a host, the host is provided with pump components, a conductive member and a retractable first interface, and the panel kit includes a power component and a monitoring component.

The power component includes a power panel and at least two pump pipeline sections, the power panel is configured to be detachably mounted to the host, the at least two pump pipeline sections are mounted to the power panel, at least one of the pump pipeline sections is configured to cooperate with at least a corresponding one of the pump components so as to drive a blood to flow along the at least one of the pump pipeline sections, and at least one of the pump pipeline sections is configured to cooperate with at least a corresponding one of the pump components so as to drive a medical liquid to flow along the at least one of the pump pipeline sections; if the power panel is mounted at a first target position of the host, the pump pipeline sections are adapted to the pump components respectively, and if the pump pipeline sections are adapted to the pump components respectively, the pump pipeline sections are capable of being driven by the pump components respectively to drive the corresponding blood or medical liquid to flow.

The monitoring component includes a monitoring panel, monitoring pipeline sections and an ESD element, the monitoring panel is configured to be detachably mounted to the host, the monitoring panel is arranged separately from the power panel such that the monitoring panel and the power panel are independent of each other and can be assembled and disassembled separately, each of the monitoring pipeline sections and the ESD element are mounted to the monitoring panel, the monitoring pipeline sections are configured to monitor parameter information of the medical liquid and/or the blood, and the parameter information at least includes a pressure parameter; at least one of the monitoring pipeline sections is provided with a second interface; if the monitoring panel is mounted at a second target position of the host, the first interface can be extended to be connected to the second interface, the ESD element is connected to the conductive member fixedly arranged on the host, or the ESD element is connected to the conductive member extended toward an exterior of the host, and if the second interface is connected to the first interface, the monitoring pipeline sections are used for the host to monitor the parameter information, and if the ESD element is connected to the conductive member, the ESD element is capable of discharging static electricity from pipeline sections of the panel kit.

According to a seventh aspect, an embodiment of the application further provides a panel kit for a blood purification device, the blood purification device includes a host, the host is provided with pump components, a conductive member and a first interface, and the panel kit includes a power component and a monitoring component.

The power component includes a power panel and at least two pump pipeline sections, the power panel is configured to be detachably mounted to the host, the at least two pump pipeline sections are mounted to the power panel, at least one of the pump pipeline sections is configured to cooperate with at least a corresponding one of the pump components so as to drive a blood to flow along the at least one of the pump pipeline sections, and at least one of the pump pipeline sections is configured to cooperate with at least a corresponding one of the pump components so as to drive a medical liquid to flow along the at least one of the pump pipeline sections; if the power panel is mounted at a first target position of the host, the pump pipeline sections are adapted to the pump components respectively, and if the pump pipeline sections are adapted to the pump components respectively, the pump pipeline sections are capable of being driven by the pump components respectively to drive the corresponding blood or medical liquid to flow.

The monitoring component includes a monitoring panel, monitoring pipeline sections and an ESD element, the monitoring panel is configured to be detachably mounted to the host, the monitoring panel is arranged separately from the power panel such that the monitoring panel and the power panel are independent of each other and can be assembled and disassembled separately, each of the monitoring pipeline sections and the ESD element are mounted to the monitoring panel, the monitoring pipeline sections are configured to monitor parameter information of the medical liquid and/or the blood, and the parameter information at least includes a pressure parameter; at least one of the monitoring pipeline sections is provided with a second interface; if the monitoring panel is mounted at a second preset position of the host, the host is capable of driving the monitoring panel to move to a second target position, such that the second interface is connected to the first interface and the ESD element is connected to the conductive member, and if the second interface is connected to the first interface, the monitoring pipeline sections are used for the host to monitor the parameter information, and if the ESD element is connected to the conductive member, the ESD element is capable of discharging static electricity from the panel kit.

According to an eighth aspect, an embodiment of the application provides a panel kit for a blood purification device, the blood purification device includes a host, the host is provided with pump components, a conductive member and a first interface, and the panel kit includes a power component and a monitoring component.

The power component includes a power panel and at least two pump pipeline sections, the power panel is configured to be detachably mounted to the host, the at least two pump pipeline sections are mounted to the power panel, at least one of the pump pipeline sections is configured to cooperate with at least a corresponding one of the pump components so as to drive a blood to flow along the at least one of the pump pipeline sections, and at least one of the pump pipeline sections is configured to cooperate with at least corresponding one of the pump components so as to drive a medical liquid to flow along the at least one of the pump pipeline sections; if the power panel is mounted at a first target position of the host, the pump pipeline sections are adapted to the pump components respectively, and if the pump pipeline sections are adapted to the pump components respectively, the pump pipeline sections are capable of being driven by the pump components respectively to drive the corresponding blood or medical liquid to flow.

The monitoring component includes a monitoring panel, monitoring pipeline sections and an ESD element, the monitoring panel is configured to be detachably mounted to the host, the monitoring panel is arranged separately from the power panel such that the monitoring panel and the power panel are independent of each other and can be assembled and disassembled separately, each of the monitoring pipeline sections and the ESD element are mounted to the monitoring panel, the monitoring pipeline sections are configured to monitor parameter information of the medical liquid and/or the blood, and the parameter information at least includes a pressure parameter; at least one of the monitoring pipeline sections is provided with a second interface; the second interface is movably arranged relative to the monitoring panel, and if the monitoring panel is mounted at a second target position of the host, the second interface is capable of being manually operated to achieve connection of the first interface to the second interface, and if the second interface is connected to the first interface, the monitoring pipeline sections are used for the host to monitor the parameter information, and if the ESD element is connected to the conductive member, the ESD element is capable of discharging static electricity from the panel kit.

In the embodiments of the application, on one hand, with at least two pump pipeline sections arranged on the power panel, a user may assist installation of the at least two pump pipeline sections by an action of mounting the power panel, therefore difficulty of mounting the at least two pump pipeline sections and a probability of mounting the at least two pump pipeline sections at wrong positions may be reduced; on the other hand, in the embodiments of the application, connection of the first interface to the second interface may be achieved by mounting the monitoring panel, thereby reducing installation operations of mounting the panel kit to the blood purification device; alternatively, connection of the second interface to the first interface may be further achieved by operating the second interface manually after the monitoring panel is mounted in place, thereby avoiding that it is difficult to mount the first interface of the panel kit in place once, thereby reducing difficulty of mounting the panel kit.

### BRIEF DESCRIPTION OF THE DRAWINGS

Technical solutions of the application and advantageous effects thereof will be apparent from detailed descriptions of specific implementations of the application below with reference to the drawings.
FIG. 1 is a schematic structural diagram of a host of a blood purification device according to an embodiment of the application.
FIG. 2 is a schematic structural diagram of cooperation and installation of the host shown in FIG. 1 and a panel kit.
FIG. 3 is a schematic diagram of a second structure of a power component of the panel kit shown in FIG. 2.
FIG. 4 is a schematic diagram of a second structure of a monitoring panel of the panel kit shown in FIG. 2.
FIG. 5 is a schematic structural diagram of a monitoring pipeline section of the panel kit shown in FIG. 2.
FIG. 6 is a schematic diagram of a second structure of a panel kit according to an embodiment of the application.
FIG. 7 is a schematic diagram of a flow path of blood in the panel kit shown in FIG. 6.
FIG. 8 is a schematic diagram of a path for a medical liquid in the panel kit shown in FIG. 6 to flow out of a first filter element.
FIG. 9 is a schematic structural diagram of a secondary membrane pressure monitoring pipeline section of a panel kit according to an embodiment of the application.
FIG. 10 is a schematic diagram of a flow path of an anticoagulant drug in the panel kit shown in FIG. 6.
FIG. 11 is a schematic diagram of flow paths of a replacement fluid and dialysate in the panel kit shown in FIG. 6.
FIG. 12 is a schematic diagram of a third structure of a panel kit according to an embodiment of the application.
FIG. 13 is a schematic diagram of a third structure of a monitoring panel of the panel kit shown in FIG. 2.

### List of reference numerals in the figures:

100. panel kit
11. power component; 111. power panel; 1111. first connection part; 1112. first region; 1113. second region; 112. pump pipeline section; 1121. blood pump pipeline section; 1122. waste liquid pressure pump pipeline section; 1123. pump pipeline section of front pump that is before blood pump; 1124. dialysate pump pipeline section; 1125. replacement fluid pump pipeline section; 1126. medical liquid pump pipeline section; 12. monitoring component; 121. monitoring panel; 1211. second connection part; 122. monitoring pipeline section; 122a. blood drawing pressure monitoring pipeline section; 122b. pressure monitoring pipeline section before filtering; 122c. waste liquid pressure monitoring pipeline section; 122d. blood leakage monitoring pipeline section; 122e. secondary membrane pressure monitoring pipeline section; 1221. second interface; 1222. pressure monitoring module; 1222a. fluid part; 1222b. gas part; 1222c. diaphragm; 1301. blood drawing flow path; 1302. blood pumping flow path; 1303. waste liquid flow path; 1304. connection flow path; 1305. blood return flow path; 1306. injection flow path; 1307. first liquid inlet flow path; 1308. second liquid inlet flow path; 1309. third liquid inlet flow path; 1310. fourth liquid inlet flow path; 1311. fifth liquid inlet flow path; 1312. sixth liquid inlet flow path; 1313. degassing pot; 1314. degassing pot monitoring interface; 1315. collection flow path; 14. electrostatic discharge (ESD) element; 15. heating component; 151. heating panel; 152. heating container; 1521. first heating container; 1522. second heating container; 153. liquid outlet flow path; 1531. first liquid outlet flow path; 1532. second liquid outlet flow path; 1533. third liquid outlet flow path;
200. host;
21. pump component; 22. first interface; 23. first driving mechanism; 231 first claw; 24. first position detection mechanism; 25. second driving mechanism; 251. second claw; 26. second position detection mechanism; 27. first pipeline switching unit; 28. second pipeline switching unit;
300. first filter element;
400. second filter element.

### DETAILED DESCRIPTION

The technical solutions in the embodiments of the application will be clearly and completely described below with reference to the drawings in the embodiments of the application. It is apparent that the described embodiments are only a part of the embodiments of the application, rather than all of the embodiments. Based on the embodiments in the application, all other embodiments obtained by those skilled in the art without paying any creative work fall within the scope of protection of the application.

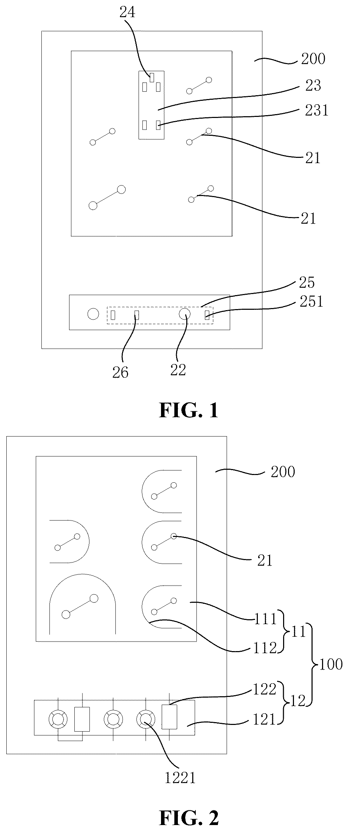

With reference to FIG. 1 and FIG. 2, according to a first aspect, an embodiment of the application provides a panel kit 100 for a blood purification device. The blood purification device may include a hemodialysis machine, a Continuous Renal Replacement Therapy (CRRT) device or the like, which is not limited in the embodiment of the application. The blood purification device may further include a host 200. The host 200 is provided with pump components 21 and a first interface 22.

The panel kit 100 may include a power component 11 and a monitoring component 12.

The power component 11 includes a power panel 111 and at least two pump pipeline sections 112. The power panel 111 is configured to be detachably mounted to the host 200, the at least two pump pipeline sections 112 are mounted to the power panel 111, at least one of the pump pipeline sections 112 is configured to cooperate with at least a corresponding one of the pump components 21 so as to drive a blood to flow along the at least one of the pump pipeline sections 112, and at least one of the pump pipeline sections 112 is configured to cooperate with at least corresponding one of the pump components 21 so as to drive a medical liquid to flow along the at least one of the pump pipeline sections 112. If the power panel 111 is mounted at a first target position of the host 200, the pump pipeline sections 112 are adapted to the pump components 21 respectively. If the pump pipeline sections 112 are adapted to the pump components 21 respectively, the pump pipeline sections 112 are capable of being driven by the pump components 21 respectively to drive the corresponding blood or medical liquid to flow. Therefore, after the power panel 111 is mounted at the first target position, it is unnecessary for a user to manually mount and fix the pump pipeline sections 112 to the pump components 21 respectively, such that installation of the power panel 111 is simpler, and a risk of mounting the at least two pump pipeline sections 112 at wrong positions may be reduced.

The monitoring component 12 includes a monitoring panel 121 and at least two monitoring pipeline sections 122. The monitoring panel 121 is configured to be detachably mounted to the host 200, the monitoring panel 121 is arranged separately from the power panel 111 such that the monitoring panel 121 and the power panel 111 can be assembled and disassembled separately, the at least two monitoring pipeline sections 122 are mounted to the monitoring panel 121, the at least two monitoring pipeline sections 122 are configured to monitor parameter information of the medical liquid and/or the blood, and the parameter information at least includes a pressure parameter. At least one of the monitoring pipeline sections 122 is provided with a second interface 1221. The monitoring component 12 is capable of being manually mounted such that the monitoring panel 121 is mounted at a second target position of the host 200 and the second interface 1221 is synchronously connected to the first interface 22. Therefore, operations of mounting the second interface 1221 independently may be reduced, and accuracy of mounting the second interface 1221 may be improved; furthermore, since the at least two monitoring pipeline sections 122 are mounted to the monitoring panel 121, installation of the monitoring panel 121 may also assist installation of at least one of other monitoring pipeline sections 122, for example, installation of the monitoring panel 121 may drive at least one of other monitoring pipeline sections 122 to be close to a corresponding position of the host 200 or directly drive at least one of other monitoring pipeline sections 122 to a corresponding position where it is connected to the host 200. As may be seen, the panel kit 100 according to the embodiment of the application may reduce difficulty of mounting pipelines in the panel kit 100 and reduce a probability of mounting the pipelines in the panel kit 100 at wrong positions.

Here, it should be noted that when it is mentioned that the monitoring component 12 is capable of being manually mounted such that the monitoring panel 121 is mounted at a second target position of the host 200 and the second interface 1221 is synchronously connected to the first interface 22, it may be understood that the operation of mounting the monitoring panel 121 at the second target position may drive the second interface 1221 to be connected to the first interface 22, thereby achieving that installation and fixation of two parts, that is, the monitoring panel 121 and the second interface 1221 are completed by one action. In this process, the operation of mounting the monitoring panel 121 at the second target position of the host 200 and the operation of connecting the second interface 1221 to the first interface 22 may be completed simultaneously, or there may be a certain time difference there-between, which is not limited in the embodiment of the application.

It should also be noted that in the embodiment of the application, when it is mentioned that the monitoring panel 121 and the power panel 111 can be assembled and disassembled separately, it means that the power panel 111 and the monitoring panel 121 are independent of each other and can be assembled and disassembled separately.

It should also be noted that the monitoring component 12 may be manually mounted once such that the monitoring panel 121 is mounted at the second target position and the second interface 1221 is synchronously connected to the first interface 22, or the monitoring component 12 may be manually mounted at least twice such that the monitoring panel 121 is mounted at the second target position and the second interface 1221 is synchronously connected to the first interface 22, which is not limited in the embodiment of the application.

It may also be understood that if the pump pipeline sections 112 and the monitoring pipeline sections 122 are integrated on the same installation panel, on one hand, a portion of the pump pipeline sections 112 and the monitoring pipeline sections 122 may not be mounted in place due to an accumulated geometric tolerance such as actual manufacturing tolerance and assembly tolerance or the like; on the other hand, the installation panel may generate severe vibration when the pump components 21 drive the pump pipeline sections 112 to operate respectively, and then the vibration of the installation panel may be transmitted to the monitoring pipeline sections 122 (such as the second interfaces 1221 of the monitoring pipeline sections 122), and may affect connection of the second interface 1221 to the first interface 22 finally, causing erroneous detection results at the monitoring pipeline sections 122. As may be seen, according to the embodiment of the application, the panel kit 100 may also have advantages of convenient installation and high accuracy of the detection result of the monitoring component 12.

The above descriptions are an overall explanation of the technical solutions in the embodiments of the application.

In some implementations, the pump pipeline sections 112 include a first flexible pipeline, the first flexible pipeline abuts against a pump head of one of the pump components 21 if the pump pipeline sections 112 are adapted to the pump components 21 respectively, such that rotation of the pump head is capable of driving the first flexible pipeline to be wound around the pump head. Therefore, in a process of mounting the power component 11, it only needs the first flexible pipeline to come into contact with the pump head of the pump component 21, and it is unnecessary for the user to manually wind the pump pipeline section 112 around the pump head of the pump component 21, thereby making installation of the pump pipeline section 112 simpler and more convenient.

For example, the pump components 21 may include a peristaltic pump, and when the first flexible pipeline comes into contact with a pump head of the peristaltic pump, the peristaltic pump drives the first flexible pipeline to be wound around the pump head first, and then the peristaltic pump continues to squeeze the first flexible pipeline by rotation of the pump head, thereby driving the medical liquid and/or the blood in the pump pipeline section 112 to flow.

Optionally, the pump pipeline sections 112 include a first flexible pipeline, the first flexible pipeline is wound around a pump head of one of the pump components 21 if the pump pipeline sections 112 are adapted to the pump components 21 respectively, such that rotation of the pump head directly drives the corresponding medical liquid and/or blood in the first flexible pipeline to flow.

It may be understood that if the pump pipeline sections 112 are adapted to the pump components 21 respectively, no matter the first flexible pipeline is directly wound around the pump head of the pump component 21 or the pump component 21 needs to operate such that the first flexible pipeline is wound around the pump head of the pump component 21, it means that after the power panel 111 is mounted at the first target position, it is unnecessary for the user to connect the pump pipeline sections 112 to the pump components 21 respectively, which may make installation of the power panel 111 simpler.

In a first mode of mounting the power component 11, if the power panel 111 is mounted at a first preset position of the host 200, the power panel 111 is capable of being driven by the host 200 to move to the first target position, such that the pump pipeline sections 112 are adapted to the pump components 21 respectively.

Therefore, for installation of the power component 11, the user only needs to mount the power panel 111 at the first preset position of the host 200, and then the host 200 automatically drives the power panel 111 to the first target position so as to achieve automatic installation of the power panel 111, such that installation of the power component 11 is simpler.

Correspondingly, if the power panel 111 is mounted at the first preset position, the pump pipeline sections 112 may not be adapted to the pump components 21 respectively. Therefore, if the power panel 111 is mounted at the first preset position, a certain gap may be reserved between the pump pipeline section 112 and the pump head, such that the pump pipeline section 112 may move toward the pump head correspondingly when the power panel 111 moves toward the first target position.

Furthermore, when the user needs to replace the panel kit 100 on the host 200 (that is, when the user needs to remove the panel kit 100 from the host 200), the host 200 may drive the power panel 111 to return from the first target position to the first preset position so as to separate the pump pipeline section 112 from the pump head of the pump component 21, such that the user may remove the power component 11 from the host 200 more conveniently.

Of course, in some other implementations, if the power panel 111 is mounted at the first preset position, the pump pipeline section 112 and the pump head of the pump component 21 may also come into contact with each other. For example, if the power panel 111 is mounted at the first preset position, the first flexible pipeline of the pump pipeline sections 112 may abut against the pump head, and the first flexible pipeline may be deformed adaptively when the power panel 111 moves toward the first target position.

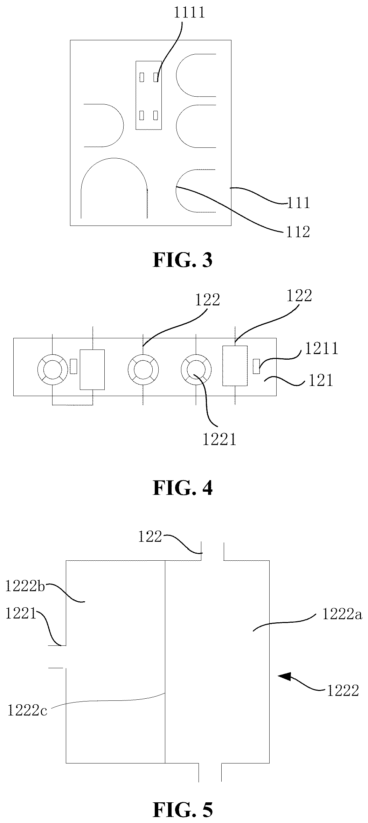

With reference to FIG. 1 and FIG. 3, in some implementations, the power panel 111 is provided with a first connection part 1111, the first connection part 1111 is configured to be connectable to a first driving mechanism 23 of the host 200, such that the first driving mechanism 23 is capable of driving the power panel 111 to move from the first preset position to the first target position.

The first connection part 1111 may be connected to the first driving mechanism 23 by clamping, screw-connecting, magnetically fixing thereto or the like, which is not limited in the embodiment of the application.

An example that the first connection part 1111 may be clamped and fixed to the first driving mechanism 23 is taken, the first driving mechanism 23 may be provided with a first claw 231, and the first claw 231 may be clamped and fixed to the first connection part 1111.

Correspondingly, the first connection part 1111 may include a first clamping hole, and the first claw 231 may be clamped into the first clamping hole. Of course, it is also possible that the first claw 231 includes at least two first clamping arms, and the first connection part 1111 is a convex structure on the power panel 111, such that the first connection part 1111 may be clamped between two first clamping arms, which is not limited in the embodiment of the application.

In some implementations, whether the power panel 111 is mounted at the first preset position and/or the first target position, is detectable by a first position detection mechanism 24 on the host 200. Therefore, position of the power panel 111 may be accurately detected by the first position detection mechanism 24, thereby determining position of the entire power component 11.

Therefore, the host 200 may determine whether the power panel 111 is mounted in place when the power panel 111 is mounted at the first preset position, based on a detection result of the first position detection mechanism 24, thereby determining whether it needs to drive the power panel 111 to move from the first preset position to the first target position by the first driving mechanism 23; and after the host 200 drives the power panel 111 to move from the first preset position to the first target position by the first driving mechanism 23, the host 200 may further determine whether the power panel 111 is mounted in place, based on the detection result of the first position detection mechanism 24, thereby avoiding abnormal installation of the power panel 111.

In some implementations, if the power panel 111 is mounted at the first preset position and/or the first target position, the power panel 111 blocks the first position detection mechanism 24 or comes into contact with the first position detection mechanism 24, such that the first position detection mechanism 24 may detect position of the power panel 111.

Exemplarily, the first position detection mechanism 24 may include a photoelectric sensor such as an infrared ranging sensor, a laser ranging sensor, a photoelectric gate or the like, such that the first position detection mechanism 24 may detect the position of the power panel 111 by detecting a distance of the power panel 111, detecting whether the power panel 111 blocks the corresponding photoelectric gate, etc.

Optionally, the first position detection mechanism 24 may further include a pressure sensor, to detect the position of the power panel 111 by a pressure applied to the first position detection mechanism 24 when the power panel 111 is mounted at the first preset position and/or the first target position.

Of course, in some other implementations, the first position detection mechanism 24 may further include a travel switch, a Hall sensor, a proximity switch or the like, which is not limited in the embodiment of the application.

It may also be understood that when the first position detection mechanism 24 is configured to detect whether the power panel 111 is mounted at the first preset position and the first target position, the first position detection mechanism 24 may include at least two first sensors, at least one of the first sensors is configured to detect whether the power panel 111 is mounted at the first preset position, and at least another one of the first sensors is configured to detect whether the power panel 111 is mounted at the first target position.

The above descriptions are introductions of the first mode of mounting the power component 11 in the embodiments of the application, and a second mode of mounting the power component 11 in the embodiments of the application will be continuously introduced below.

In a second mode of mounting the power component 11, the power component 11 is capable of being manually mounted such that the power panel 111 is mounted at the first target position and the pump pipeline sections 112 are synchronously adapted to the pump components 21 respectively. Therefore, after the power panel 111 is mounted at the first target position, it is unnecessary for the user to manually mount the pump pipeline section 112 to the pump head, which may reduce difficulty of mounting the power component 11 by the user.

Correspondingly, whether the power panel 111 is mounted at the first target position, is detectable by a first position detection mechanism 24 on the host 200. Therefore, the position of the power panel 111 may be accurately detected by the first position detection mechanism 24, thereby determining the position of the entire power component 11.

Specific structures of the first position detection mechanism 24 are not elaborated here.

In some implementations, the power panel 111 may be provided with a first connection part 1111, the first connection part 1111 is configured to be connectable to the host 200 so as to achieve that the power panel 111 is detachably mounted at the first target position.

For example, the host 200 may be fixedly provided with a first claw 231, and specific structures of the first claw 231 and the first connection part 1111 are not elaborated here.

The above descriptions are some introductions of the second mode of mounting the power component 11 in the embodiments of the application, and the technical solutions in the embodiments of the application will be continuously introduced below with reference to the monitoring component 12 in the embodiments of the application.

With continuous reference to FIG. 1 and FIG. 4, in some implementations, the monitoring panel 121 is further provided with a second connection part 1211, the second connection part 1211 is configured to be clamped to a second claw 251 of the host 200, so as to achieve that the monitoring panel 121 is detachably mounted to the host 200.

For example, the second connection part 1211 may include a second clamping hole, and the second claw 251 may be clamped into the second clamping hole. Of course, it is also possible that the second claw 251 includes at least two second clamping arms, and the second connection part 1211 is a convex structure on the monitoring panel 121, such that the second connection part 1211 may be clamped between two second clamping arms, which is not limited in the embodiment of the application.

In some implementations, whether the monitoring panel 121 is mounted at the second target position, is detectable by a second position detection mechanism 26 on the host 200. Therefore, position of the monitoring panel 121 may be accurately detected by the second position detection mechanism 26, thereby determining position of the entire monitoring component 12. For example, the host 200 may determine whether the monitoring component 12 is mounted in place or the like, to avoid erroneous detection results caused by not mounting the monitoring component 12 in place.

In some implementations, if the monitoring panel 121 is mounted at the second target position, the monitoring panel 121 blocks the second position detection mechanism 26 or comes into contact with the second position detection mechanism 26, such that the second position detection mechanism 26 may detect position of the monitoring panel 121.

Exemplarily, the second position detection mechanism 26 may include a photoelectric sensor such as an infrared ranging sensor, a laser ranging sensor, a photoelectric gate or the like, such that the second position detection mechanism 26 may detect the position of the monitoring panel 121 by detecting a distance of the monitoring panel 121, detecting whether the monitoring panel 121 blocks the corresponding photoelectric gate, etc.

Optionally, the second position detection mechanism 26 may further include a pressure sensor, to detect the position of the monitoring panel 121 by a pressure applied to the second position detection mechanism 26 when the monitoring panel 121 is mounted at the second target position.

Of course, in some other implementations, the second position detection mechanism 26 may further include a travel switch, a Hall sensor, a proximity switch or the like, which is not limited in the embodiment of the application.

It may also be understood that when the second position detection mechanism 26 is configured to detect whether the monitoring panel 121 is mounted at the second preset position and the second target position, the second position detection mechanism 26 may include at least two second sensors, at least one of the second sensors is configured to detect whether the monitoring panel 121 is mounted at the second preset position, and at least another one of the second sensors is configured to detect whether the monitoring panel 121 is mounted at the second target position.

In some implementations, the panel kit 100 further includes a second flexible pipeline, the second flexible pipeline is connected between at least one of the pump pipeline sections 112 and at least one of the monitoring pipeline sections 122.

It may be understood that when the pump components 21 of the host 200 drive the pump pipeline sections 112 to operate respectively, the pump components 21 not only cause the power panel 111 to vibrate but also pull the pump pipeline sections 112 respectively. Therefore, on a basis that the power panel 111 is separated from the monitoring panel 121 to prevent vibration of the power panel 111 from transmitting to the monitoring panel 121, when the pump pipeline sections 112 are pulled, the second flexible pipeline may be adaptively deformed to prevent the pump pipeline sections 112 from pulling the monitoring pipeline sections 122 connected thereto, thereby preventing the second interface 1221 from loosening and falling off by interference of the monitoring panel 121 and/or the second flexible pipeline connected thereto, and accuracy of the detection result of the blood purification device may be improved finally.

In some implementations, flow paths connected between the pump pipeline sections 112 and the monitoring pipeline sections 122 are hoses.

In some implementations, the second interface 1221 is fixedly arranged relative to the monitoring panel 121. Therefore, in a process of mounting the monitoring panel 121, it may avoid that the second interface 1221 moves relative to the monitoring panel 121, and thus it may avoid that the second interface 1221 collides with the first interface 22 and cannot be mounted in place. Of course, when the second interface 1221 is prevented from moving relative to the monitoring panel 121, it may also avoid that the second interface 1221 is shaken during operation of the blood purification device to cause the second interface 1221 to loosen and fall off relative to the first interface 22, therefore accuracy and reliability of the detection result of the blood purification device may be improved.

In some implementations, the second interface 1221 is movably arranged relative to the monitoring panel 121.

For example, in the process of mounting the monitoring panel 121 at the second target position, the second interface 1221 is movable relative to the monitoring panel 121 to compensate for a tolerance of at least one of the second interface 1221, the monitoring panel 121, and the host 200.

Therefore, when the second interface 1221 is movable relative to the monitoring panel 121, it may avoid that the second interface 1221 cannot be connected to the first interface 22 due to a geometric tolerance accumulated in processes of manufacturing and assembling each part of the second interface 1221, the monitoring panel 121 and the host 200. Furthermore, the second interface 1221 is controlled to be movable in a small range only to compensate for the tolerance, it may also avoid that there is an excessive movement amplitude of the second interface 1221 relative to the monitoring panel 121, and thus it may avoid that the second interface 1221 collides with the first interface 22 and cannot be mounted in place. Of course, when the excessive movement amplitude of the second interface 1221 relative to the monitoring panel 121 is avoided, it may also avoid that the second interface 1221 is shaken during operation of the blood purification device to cause the second interface 1221 to loosen and fall off, therefore accuracy and reliability of the detection result of the blood purification device may be improved.

In some implementations, the second interface 1221 is non-detachably connected to a corresponding one of the monitoring pipeline sections 122. For example, the second interface 1221 may be connected to the corresponding monitoring pipeline section 122 by bonding, integrally molding, fusion-connecting, welding thereto or the like, therefore situations where the second interface 1221 is loosened or even fallen off or the like to cause an inaccurate detection result of the host 200, may be avoided.

Optionally, in some other implementations, the second interface 1221 is detachably connected to the corresponding monitoring pipeline section 122. For example, the second interface 1221 is connected to the corresponding monitoring pipeline section 122 by screw-connecting, clamping, magnetically fixing thereto or the like, such that appropriate second interfaces 1221 may be replaced based on variations of models of the first interfaces 22 of the host 200.

In some implementations, the second interface 1221 is connected to the corresponding monitoring pipeline section 122 by a connector. The connector may be a Luer connector or other forms of connectors, which is not limited in the embodiment of the application.

In some implementations, the monitoring pipeline sections 122 are non-detachably connected to the monitoring panel 121. For example, the monitoring pipeline sections 122 may be connected to the monitoring panel 121 by bonding, integrally molding, fusion-connecting, welding thereto or the like, therefore situations where the monitoring pipeline sections 122 are loosened or even fallen off or the like to cause an inaccurate detection result of the host 200, may be avoided.

Optionally, the monitoring pipeline sections 122 may also be detachably connected to the monitoring panel 121. For example, the monitoring pipeline sections 122 may be connected to the monitoring panel 121 by screw-connecting, clamping, magnetically fixing thereto or the like, such that appropriate monitoring pipeline sections 122 or monitoring panels 121 may be replaced based on variations of the host 200.

With continuous reference to FIG. 5, in some implementations, at least one of the monitoring pipeline sections 122 includes a pressure monitoring module 1222, the pressure monitoring module 1222 includes a fluid part 1222a, a gas part 1222b, a diaphragm 1222c and the second interface 1221. A side cavity for fluid defined at least partially by the fluid part 1222a is separated from a side cavity for gas defined at least partially by the gas part 1222b through the diaphragm 1222c, the side cavity for fluid is used for the blood or the medical liquid to flow there-through, the second interface 1221 is fluidly communicated with the side cavity for gas, and if the second interface 1221 is connected to the first interface 22, the monitoring pipeline sections 122 are used for the host 200 to monitor a pressure parameter of the medical liquid or the blood flowing through the side cavity for fluid.

Therefore, when the blood or the medical liquid flows through the side cavity for fluid, the blood or the medical liquid may squeeze the diaphragm 1222c to cause a gas pressure in the side cavity for gas to change, and then the pressure parameter of the corresponding blood or medical liquid may be measured by docking the first interface 22 on the host 200 with the second interface 1221 to measure the gas pressure in the side cavity for gas, such that it is unnecessary for the first interface 22 to come into contact with the corresponding blood or medical liquid, thereby avoiding that the blood or the medical liquid is contaminated.

Of course, in some other implementations, it is also possible that at least one of the monitoring pipeline sections 122 is adapted to a contact pressure sensor on the host 200. Therefore, the contact pressure sensor on the host 200 may directly abut against the diaphragm 1222c or other flexible regions of the monitoring pipeline section 122, to monitor the pressure parameter of the corresponding blood or medical liquid, which is not limited in the embodiment of the application.

In some implementations, at least two of the monitoring pipeline sections 122 are provided with the second interfaces 1221 respectively, and the first interface 22 includes at least two first interfaces, and the second interfaces 1221 are configured to be connected to the corresponding first interfaces 22 on the host 200 respectively, such that the host 200 is capable of monitoring the pressure parameter of the corresponding blood or medical liquid through the monitoring pipeline sections 122. Therefore, connection of at least two second interfaces 1221 for measuring the pressure parameter may be achieved through the monitoring panel 121, such that installation of the monitoring component 12 is more convenient.

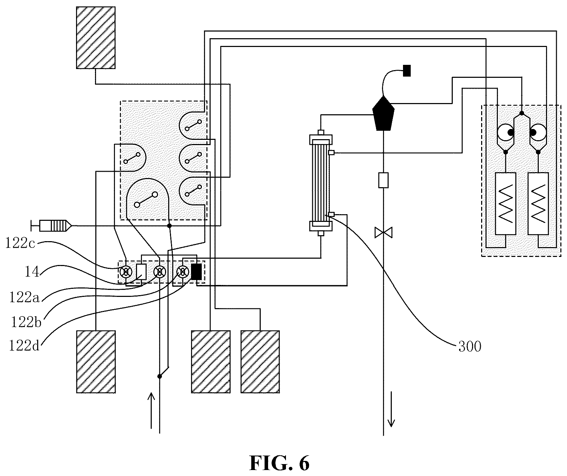

With continuous reference to FIG. 6, in some implementations, the blood purification device further includes a first filter element 300. The first filter element 300 is configured to filter the blood flowing through the pump pipeline sections 112.

The first filter element 300 may be arranged on the host 200, and after the panel kit 100 is mounted to the host 200, the panel kit 100 is connected to the first filter element 300 on the host 200. Alternatively, the first filter element 300 may be arranged on the panel kit 100, and the first filter element 300 is fixed to the host 200 in a process of mounting the panel kit 100 to the host 200, which is not limited in the embodiment of the application.

In some implementations, if the second interfaces 1221 are connected to the first interfaces 22 respectively, the monitoring pipeline sections 122 are used for the host 200 to monitor the pressure parameter of the corresponding blood or medical liquid; the at least two monitoring pipeline sections 122 further include a blood leakage monitoring pipeline section 122d, the blood leakage monitoring pipeline section 122d is configured to cooperate with a blood leakage monitoring module on the host 200, so as to monitor whether the blood at one side of a filtration membrane of the first filter element 300 permeates to the other side of the filtration membrane. Therefore, connection of at least one second interface 1221 for measuring the pressure parameter and installation of the blood leakage monitoring pipeline section 122d may be achieved through the monitoring panel 121, such that installation of the monitoring component 12 is more convenient.

In some implementations, the monitoring pipeline sections 122 include at least one of a blood drawing pressure monitoring pipeline section 122a, a pressure monitoring pipeline section before filtering 122b, a waste liquid pressure monitoring pipeline section 122c and a blood leakage monitoring pipeline section 122d.

The blood drawing pressure monitoring pipeline section 122a is configured to measure the pressure parameter of the blood drawn out of a patient's blood vessel (hereinafter, referred to as a blood drawing pressure simply). Therefore, the host 200 may determine whether the blood is drawn out of the patient's blood vessel normally, by monitoring the blood drawing pressure.

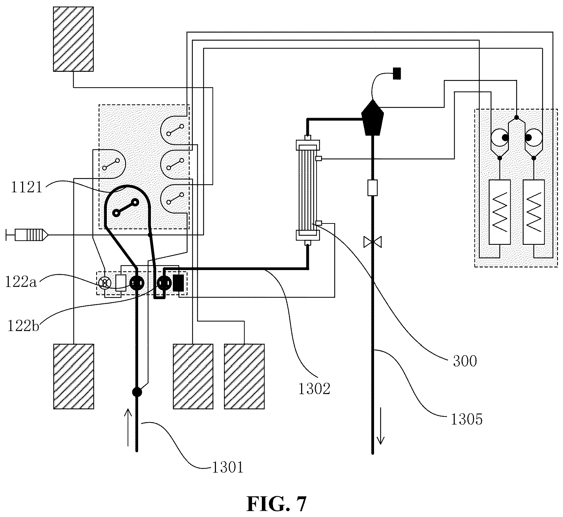

With continuous reference to FIG. 7, for example, the pump pipeline sections 112 include a blood pump pipeline section 1121, the blood pump pipeline section 1121 is configured to cooperate with one of the pump components 21 so as to drive the blood to flow. The panel kit 100 further includes a blood drawing flow path 1301, the blood drawing flow path 1301 is configured to connect an input of the blood pump pipeline section 1121 to the patient's blood vessel, and the blood drawing flow path 1301 is provided with the blood drawing pressure monitoring pipeline section 122a.

Therefore, when the pump component 21 operates, the blood pump pipeline section 1121 may drive the blood from the patient's blood vessel to flow into the blood drawing flow path 1301 and then flow through the blood drawing pressure monitoring pipeline section 122a when the blood travels along the blood drawing flow path 1301, such that the blood drawing pressure is monitored in the blood drawing pressure monitoring pipeline section 122a.

The pressure monitoring pipeline section before filtering 122b is configured to measure a pressure parameter of the blood flowing from the pump pipeline sections 112 into the first filter element 300 (hereinafter, referred to as a pressure before filtering simply). Therefore, whether the blood may flow into the first filter element 300 normally may be determined by measuring the pressure before filtering. For example, when the pressure before filtering is too high, there may be a situation where blockage, coagulation or the like occurs to the first filter element 300.

Exemplarily, the pump pipeline sections 112 include a blood pump pipeline section 1121, the blood pump pipeline section 1121 is configured to cooperate with one of the pump components 21 so as to drive the blood to flow, the panel kit 100 further includes a blood pumping flow path 1302, the blood pumping flow path 1302 is connected to an output of the blood pump pipeline section 1121 and a blood input of the first filter element 300 respectively, and the blood pumping flow path 1302 is provided with the pressure monitoring pipeline section before filtering 122b.

Therefore, when the pump component 21 operates, the blood from the patient's blood vessel may flow into the first filter element 300 by passing through the blood drawing flow path 1301, the blood pump pipeline section 1121 and the blood pumping flow path 1302 sequentially, such that the blood flowing into the first filter element 300 may flow through the pressure monitoring pipeline section before filtering 122b.

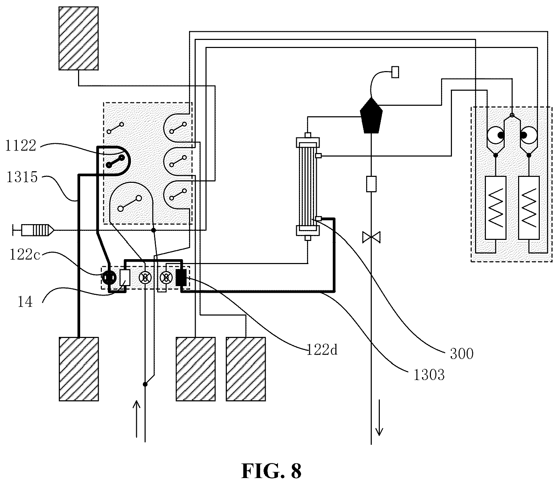

With continuous reference to FIG. 8, the waste liquid pressure monitoring pipeline section 122c is configured to measure a pressure parameter of the medical liquid discharged from the first filter element 300 (hereinafter, referred to as a waste liquid pressure simply). Therefore, whether the filtration membrane of the first filter element 300 is blocked may be determined by measuring the waste liquid pressure.

Exemplarily, the pump pipeline sections 112 include a waste liquid pressure pump pipeline section 1122, the waste liquid pressure pump pipeline section 1122 is configured to cooperate with one of the pump components 21 so as to drive the medical liquid to discharge from the first filter element 300, the panel kit 100 further includes a waste liquid flow path 1303, the waste liquid flow path 1303 is connected to a medical liquid output of the first filter element 300 and an input of the waste liquid pressure pump pipeline section 1122 respectively, and the waste liquid flow path 1303 is provided with the waste liquid pressure monitoring pipeline section 122c.

Therefore, when the pump component 21 operates, the medical liquid in the first filter element 300 may flow along the waste liquid flow path 1303 to the waste liquid pressure pump pipeline section 1122, to flow through the waste liquid pressure monitoring pipeline section 122c.

It should be noted here that compared to arranging the waste liquid pressure monitoring pipeline section 122c downstream of the waste liquid pressure pump pipeline section 1122, in the embodiment of the application, the waste liquid pressure monitoring pipeline section 122c is arranged between the waste liquid pressure pump pipeline section 1122 and the first filter element 300, which may avoid that other faults downstream of the waste liquid pressure pump pipeline section 1122 interfere with the waste liquid pressure monitoring pipeline section 122c monitoring whether the filtration membrane of the first filter element 300 is blocked.

The blood leakage monitoring pipeline section 122d is configured to cooperate with a blood leakage monitoring module of the host 200, so as to monitor whether the blood at one side of a filtration membrane of the first filter element 300 permeates to the other side of the filtration membrane. Therefore, the blood leakage monitoring module may determine whether the filtration membrane of the filter is damaged, causing the blood to permeate the filtration membrane.

Exemplarily, the pump pipeline sections 112 include a waste liquid pressure pump pipeline section 1122, the waste liquid pressure pump pipeline section 1122 is configured to cooperate with one of the pump components 21 so as to drive the medical liquid to discharge from the first filter element 300. The panel kit 100 further includes a waste liquid flow path 1303 and a collection flow path 1315, the waste liquid flow path 1303 is connected to a medical liquid output of the first filter element 300 and an input of the waste liquid pressure pump pipeline section 1122 respectively, an input of the collection flow path 1315 is connected to an output of the waste liquid pressure pump pipeline section 1122, and the blood leakage monitoring pipeline section 122d is arranged in the waste liquid flow path 1303 or the collection flow path 1315.

Therefore, when the pump component 21 operates, the medical liquid in the first filter element 300 may be collected by an external waste liquid collection container after flowing through the waste liquid flow path 1303, the waste liquid pressure pump pipeline section 1122 and the collection flow path 1315 sequentially. In this process, monitoring may be performed through the blood leakage monitoring pipeline section 122d on the waste liquid flow path 1303 or the collection flow path 1315.

In some implementations, the blood leakage monitoring module on the host 200 may include a photoelectric sensor such as a color sensor, to determine whether there is a blood composition by monitoring color of the medical liquid discharged from the first filter element 300.

Correspondingly, at least a portion of the blood leakage monitoring pipeline section 122d may be made of a light-shielding material, or the blood leakage monitoring pipeline section 122d may be provided with a light-shielding member to prevent ambient light from affecting a detection result of the blood leakage monitoring module.

Of course, the blood leakage monitoring module may be any other modules for detecting blood compositions, which is not limited in the embodiment of the application.

It may also be understood that an input of the blood leakage monitoring pipeline section 122d may face downward, such that the medical liquid flows through the blood leakage monitoring pipeline section 122d from bottom to top, thereby avoiding that bubbles are accumulated when the medical liquid flows through the blood leakage monitoring pipeline section 122d from top to bottom and affect the detection result of the blood leakage monitoring module. Of course, in some other implementations, the input of the blood leakage monitoring pipeline section 122d may also face other directions, which is not limited in the embodiment of the application.

Similarly, an input of at least one of the monitoring pipeline sections 122 may face downward, such that the corresponding medical liquid or blood flows through the monitoring pipeline section 122 from bottom to top, thereby avoiding that bubbles are accumulated in the monitoring pipeline section 122 and affect the detection result.

Optionally, in some other implementations, the panel kit 100 includes a waste liquid branch, the waste liquid branch is configured to be communicated with the medical liquid output of the first filter element 300. The waste liquid branch is provided with the blood leakage monitoring pipeline section 122d, the waste liquid branch may allow the medical liquid flowing out of the first filter element 300 to flow through the blood leakage monitoring pipeline section 122d. The blood leakage monitoring pipeline section is configured to cooperate with a blood leakage monitoring module on the host, so as to monitor whether the blood at one side of a filtration membrane of the first filter element 300 permeates to the other side of the filtration membrane.

For example, the waste liquid branch includes the waste liquid flow path 1303 and the collection flow path 1315. Therefore, the blood leakage monitoring pipeline section 122d may be arranged in the waste liquid flow path 1303 or the collection flow path 1315, which is not limited in the embodiment of the application.

In some implementations, the waste liquid branch may allow the medical liquid flowing out of the first filter element 300 to flow through the blood leakage monitoring pipeline section 122d from bottom to top, thereby avoiding that bubbles are accumulated in the blood leakage monitoring pipeline section 122d and affect the detection result.

It may also be understood that the blood leakage monitoring pipeline section 122d may be arranged on the monitoring panel 121, to be used as one of the monitoring pipeline sections 122; or, the blood leakage monitoring pipeline section 122d may not be arranged on the monitoring panel 121, for example, the blood leakage monitoring pipeline section 122d may be arranged between the power panel 111 and the monitoring panel 121, which is not limited in the embodiment of the application.

Exemplarily, the blood leakage monitoring pipeline section 122d may be arranged in the waste liquid flow path 1303 and located between the input of the waste liquid pressure pump pipeline section 1122 and an output of the waste liquid pressure monitoring pipeline section 122c.

Therefore, on one hand, it may avoid that it needs to reserve a large space on the monitoring panel 121 to mount the blood leakage monitoring pipeline section 122d, therefore it is beneficial for miniaturization design of the monitoring panel 121; on the other hand, it may also reduce the number of the monitoring pipeline sections 122 on the monitoring panel 121, such that it is unnecessary for pipelines of the panel kit 100 to bend multiple times on the monitoring panel 121 to configure more monitoring pipeline sections 122, therefore it may not only reduce difficulty of manufacturing the panel kit 100, but also allow the corresponding blood or medical liquid to flow more smoothly in the panel kit 100.

Furthermore, when an input of the waste liquid pressure monitoring pipeline section 122c also faces downward, the input of the blood leakage monitoring pipeline section 122d may also face downward, to be connected to the output of the waste liquid pressure monitoring pipeline section 122c arranged to face upward, thereby avoiding that bubbles are accumulated when the medical liquid flows through the blood leakage monitoring pipeline section 122d from top to bottom and affect the detection result of the blood leakage monitoring module.

In some implementations, the monitoring component 12 includes at least two monitoring components, any two of the monitoring components 12 are arranged separately from each other, such that each of the monitoring components 12 can be assembled and disassembled separately, and each of the monitoring components 12 is provided with at least two of the blood leakage monitoring pipeline section 122d, the blood drawing pressure monitoring pipeline section 122a, the pressure monitoring pipeline section before filtering 122b and the waste liquid pressure monitoring pipeline section 122c. Therefore, with the at least two monitoring components 12, the panel kit 100 may be adapted to installation requirements of more different types of hosts 200.

Exemplarily, one of the monitoring components 12 may be provided with the blood drawing pressure monitoring pipeline section 122a and the pressure monitoring pipeline section before filtering 122b, and the other one of the monitoring components 12 may be provided with the blood leakage monitoring pipeline section 122d and the waste liquid pressure monitoring pipeline section 122c, which is not limited in the embodiment of the application.

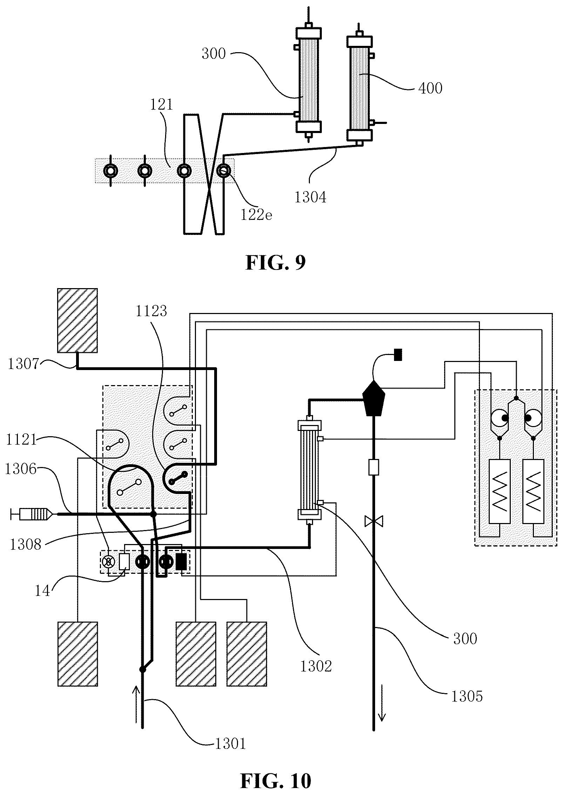

With continuous reference to FIG. 9, in some implementations, the blood purification device further includes a second filter element 400, the second filter element 400 is configured to re-filter the blood flowing through the pump pipeline sections 112, a blood input of the second filter element 400 is fluidly communicated with a blood output of the first filter element 300 so as to re-filter the blood flowing out of the blood output of the first filter element 300. The monitoring pipeline sections 122 further include a secondary membrane pressure monitoring pipeline section 122e, the secondary membrane pressure monitoring pipeline section 122e is configured to fluidly communicate the first filter element 300 with the second filter element 400, and the secondary membrane pressure monitoring pipeline section 122e is configured to monitor the pressure parameter of the blood.

For example, the panel kit 100 may further include a connection flow path 1304, the connection flow path 1304 is communicated with the blood output of the first filter element 300 and the blood input of the second filter element 400 respectively, and the secondary membrane pressure monitoring pipeline section 122e is arranged in the connection flow path 1304.

Correspondingly, when the monitoring component 12 includes at least two monitoring components, any two of the monitoring components 12 are arranged separately from each other, such that each of the monitoring components 12 can be assembled and disassembled separately, then each of the monitoring components 12 is provided with at least two of the blood leakage monitoring pipeline section 122d, the blood drawing pressure monitoring pipeline section 122a, the pressure monitoring pipeline section before filtering 122b, the waste liquid pressure monitoring pipeline section 122c and the secondary membrane pressure monitoring pipeline section 122e. Therefore, with the at least two monitoring components 12, the panel kit 100 may be adapted to installation requirements of more different types of hosts 200.

In some implementations, the input of the collection flow path 1315 is connected to the output of the waste liquid pressure pump pipeline section 1122, and an output of the collection flow path 1315 is configured to be connected to the secondary membrane pressure monitoring pipeline section 122e. Therefore, the medical liquid in the first filter element 300 may flow along the waste liquid flow path 1303, the waste liquid pressure pump pipeline section 1122 and the collection flow path 1315 sequentially, and then flow into the secondary membrane pressure monitoring pipeline section 122e.

With continuous reference to FIG. 10, in some implementations, the panel kit 100 further includes an electrostatic discharge (ESD) element 14, the ESD element 14 is configured to cooperate with the host 200 so as to discharge static electricity from the panel kit 100. It may be understood that static electricity may also be generated when the pump components 21 drive the pump pipeline sections 112 to move respectively, then the static electricity is discharged by the ESD element 14, to avoid interference with other devices used simultaneously, such as an electrocardiograph (ECG) monitor.

In some implementations, the ESD element 14 is arranged on at least one of the power panel 111 and the monitoring panel 121, such that with the ESD element 14 integrated into the corresponding power panel 111 and/or monitoring panel 121, adaptation of the ESD element 14 to the host 200 may be achieved synchronously through installation of the corresponding power panel 111 and/or monitoring panel 121, thereby facilitating installation of the panel kit 100.

Of course, in some other implementations, it is also possible that the ESD element 14 is not arranged on the power panel 111 and is not arranged on the monitoring panel 121, which is not limited in the embodiment of the application.

In some implementations, the host 200 is provided with a conductive member, and the ESD element 14 is configured to be connected to the conductive member so as to discharge static electricity. For example, the ESD element 14 may come into contact with the conductive member to form electric connection, such that the ESD element 14 is grounded through the conductive member.

In some implementations, a portion of pipeline sections of the panel kit 100 is made of a conductive material to form the ESD element 14. Of course, the ESD element 14 may also be a conductive element connected to one of the pipeline sections of the panel kit 100, which is not limited in the embodiment of the application.

In some implementations, the pump pipeline sections 112 include the blood pump pipeline section 1121. The panel kit 100 further includes the blood drawing flow path 1301, the blood pumping flow path 1302 and a blood return flow path 1305. An input of the blood drawing flow path 1301 is configured to be connected to a blood drawing site of the patient's blood vessel, and an output of the blood drawing flow path 1301 is connected to the input of the blood pump pipeline section 1121. An input of the blood pumping flow path 1302 is connected to the output of the blood pump pipeline section 1121, and an output of the blood pumping flow path 1302 is configured to be connected to the blood input of the first filter element 300. An input of the blood return flow path 1305 is configured to be connected to a blood output of the first filter element 300, and an output of the blood return flow path 1305 is configured to be connected to a blood return site of the patient's blood vessel.

Therefore, during operation of the blood purification device, when the pump component 21 of the host 200 operates, the blood pump pipeline section 1121 drives the blood to flow through the blood drawing flow path 1301, the blood pump pipeline section 1121, the blood pumping flow path 1302, the first filter element 300 and the blood return flow path 1305 sequentially from the blood drawing site of the patient's blood vessel, and then flow back to the patient's blood vessel from the blood return site of the patient's blood vessel, thereby achieving treatment of the patient with extracorporeal blood circulation.

In some implementations, the panel kit 100 further includes an injection flow path 1306, an input of the injection flow path 1306 is configured to be connected to an anticoagulant drug syringe pump, and an output of the injection flow path 1306 is connected to the blood drawing flow path 1301 or the blood pumping flow path 1302. Therefore, the anticoagulant drug syringe pump provided on the host 200 may inject the anticoagulant drug into the blood in the blood drawing flow path 1301 or the blood pumping flow path 1302 through the injection flow path 1306, to avoid coagulation of the blood in the blood drawing flow path 1301 or the blood pumping flow path 1302.

The anticoagulant drug may include heparin, citric acid or the like, which is not limited in the embodiment of the application.

It may also be understood that when the injection flow path 1306 is connected to the blood pumping flow path 1302, it may avoid that the pump component 21 generates a large negative pressure in the blood drawing flow path 1301 when the pump component 21 operates, to suck the anticoagulant drug out of the anticoagulant drug syringe pump to a certain extent.

In some implementations, the pump pipeline sections 112 further include a pump pipeline section of a front pump that is before the blood pump 1123, the panel kit 100 includes a first liquid inlet flow path 1307 and a second liquid inlet flow path 1308, the first liquid inlet flow path 1307, the pump pipeline section of the front pump 1123 and the second liquid inlet flow path 1308 are connected sequentially, an input of the first liquid inlet flow path 1307 is at least configured to be connected to an anticoagulant drug supply container, and an output of the second liquid inlet flow path 1308 is connected to the blood drawing flow path 1301, the blood pumping flow path 1302 or the blood return flow path 1305. Therefore, the host 200 may drive the pump pipeline section of the front pump 1123 through the pump component 21 to allow the anticoagulant drug in the anticoagulant drug supply container to flow into the blood drawing flow path 1301, the blood pumping flow path 1302 or the blood return flow path 1305 after passing through the first inlet flow path 1307, the pump pipeline section of the front pump 1123 and the second inlet flow path 1308 sequentially, so as to avoid coagulation of the blood in the blood drawing flow path 1301, the blood pumping flow path 1302 or the blood return flow path 1305.

It may be understood that when the panel kit 100 is provided with the injection flow path 1306, the first liquid inlet flow path 1307, the pump pipeline section of the front pump 1123 and the second liquid inlet flow path 1308 simultaneously, it is possible that the anticoagulant drug syringe pump connected to the injection flow path 1306 and the anticoagulant drug supply container connected to the first liquid inlet flow path 1307 are configured to supply different anticoagulant drugs. For example, the anticoagulant drug syringe pump is configured to supply heparin, and the anticoagulant drug supply container is configured to supply citric acid, to meet different anticoagulant requirements during extracorporeal blood purification.

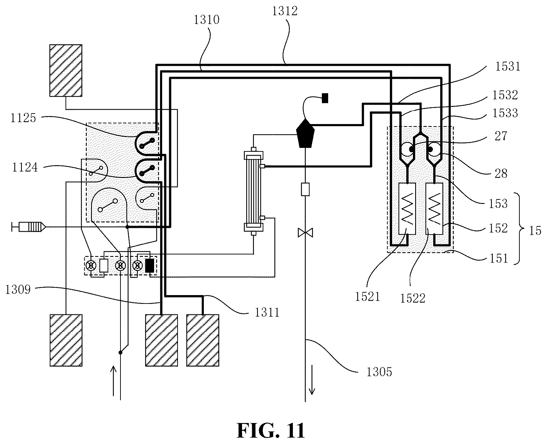

With continuous reference to FIG. 11, in some implementations, the pump pipeline sections 112 include a dialysate pump pipeline section 1124, and the panel kit 100 further includes a third liquid inlet flow path 1309 and a fourth liquid inlet flow path 1310, the third liquid inlet flow path 1309, the dialysate pump pipeline section 1124 and the fourth liquid inlet flow path 1310 are connected sequentially, an input of the third liquid inlet flow path 1309 is configured to be connected to a dialysate supply container, and an output of the fourth liquid inlet flow path 1310 is configured to be connected to a medical liquid input of the first filter element 300.

Therefore, when the pump component 21 on the host 200 operates, the pump component 21 may drive dialysate in the dialysate supply container through the dialysate pump pipeline section 1124, to flow into the first filter element 300 by passing through the third liquid inlet flow path 1309, the dialysate pump pipeline section 1124, the fourth liquid inlet flow path 1310 and the medical liquid input of the first filter element 300 sequentially.

In some implementations, the pump pipeline sections 112 further include a replacement fluid pump pipeline section 1125, the panel kit 100 further includes a fifth liquid inlet flow path 1311 and a sixth liquid inlet flow path 1312, the fifth liquid inlet flow path 1311, the replacement fluid pump pipeline section 1125 and the sixth liquid inlet flow path 1312 are connected sequentially, an input of the fifth liquid inlet flow path 1311 is configured to be connected to a replacement fluid supply container, and an output of the sixth liquid inlet flow path 1312 is configured to be connected to the blood drawing flow path 1301, the blood pumping flow path 1302 or the blood return flow path 1305.

Therefore, when the pump component 21 on the host 200 operates, the pump component 21 may drive a replacement fluid in the replacement fluid supply container through the replacement fluid pump pipeline section 1125, to flow into the blood drawing flow path 1301, the blood pumping flow path 1302 or the blood return flow path 1305 after passing through the fifth liquid inlet flow path 1311, the replacement fluid pump pipeline section 1125 and the sixth liquid inlet flow path 1312 sequentially.

The replacement fluid may include a commercially available replacement fluid, such as an online replacement fluid produced on-line by a hemodiafiltration (HDF) machine, a manually prepared replacement fluid or the like, which is not limited in the embodiment of the application.

In some implementations, the panel kit 100 further includes a heating component 15, the heating component 15 is configured to be mounted on a heating region of the host 200 such that the medical liquid and/or the blood in the heating component 15 is heated by a heating unit of the host 200. Therefore, heating requirements of the medical liquid and/or the blood may be met. For example, the medical liquid may be heated to prevent the medical liquid with a too low temperature from affecting the blood, for example, the medical liquid with a too low temperature causes a too low temperature of the blood flowing back to a human body, to cause discomfort of the human.

The heating component 15 may include a heating panel 151 and a heating container 152. The heating panel 151 is configured to be detachably mounted on the heating region of the host 200. The heating container 152 is mounted to the heating panel 151 so as to be mounted on the heating region by the heating panel 151, such that the heating container 152 may be conveniently mounted to the host 200 by the heating panel 151.

It may be understood that the heating container 152 may include a coil-type heating container, a bladder-type heating container, a bottle-type heating container, a bag-type heating container or the like, which is not limited in the embodiment of the application.

The heating component 15 may further include a liquid outlet flow path 153. An input of the liquid outlet flow path 153 is communicated with an output of the heating container 152. The input of the liquid outlet flow path 153 may be mounted to the heating panel 151. Therefore, the heating container 152, the input of the liquid outlet flow path 153 or the like may be conveniently mounted to the host 200 through the heating panel 151.

In some implementations, the pump pipeline sections 112 include the dialysate pump pipeline section 1124, the heating container 152 includes a first heating container 1521, an input connected to the first heating container 1521 is connected to the output of the fourth liquid inlet flow path 1310, the liquid outlet flow path 153 connected to the first heating container 1521 includes a first liquid outlet flow path 1531 and a second liquid outlet flow path 1532, the first liquid outlet flow path 1531 is connected to the blood return flow path 1305, and the second liquid outlet flow path 1532 is connected to the medical liquid input of the first filter element 300.

Therefore, during actual usage, the input of the third liquid inlet flow path 1309 may be selectively connected to the dialysate supply container or the replacement fluid supply container, such that the blood purification device may meet more different use requirements.

For example, when the input of the third liquid inlet flow path 1309 is connected to the dialysate supply container, the first liquid outlet flow path 1531 may be controlled to open and the second liquid outlet flow path 1532 may be controlled to be in communication by a first pipeline switching unit 27 on the host 200, such that the dialysate in the dialysate supply container flows into the first filter element 300 after passing through the third liquid inlet flow path 1309, the dialysate pump pipeline section 1124, the fourth liquid inlet flow path 1310, the first heating container 1521, the second liquid outlet flow path 1532 and the medical liquid input of the first filter element 300 sequentially.

Alternatively, when the input of the third liquid inlet flow path 1309 is connected to the replacement fluid supply container, the first liquid outlet flow path 1531 may be controlled to be in communication and the second liquid outlet flow path 1532 may be controlled to open by the first pipeline switching unit 27 on the host 200, such that the replacement fluid in the replacement fluid supply container flows into the blood return flow path 1305 after passing through the third liquid inlet flow path 1309, the dialysate pump pipeline section 1124, the fourth liquid inlet flow path 1310, the first heating container 1521 and the first liquid outlet flow path 1531 sequentially.

In some implementations, the pump pipeline sections 112 include the replacement fluid pump pipeline section 1125, the heating container 152 includes a second heating container 1522, an input connected to the second heating container 1522 is connected to the output of the sixth liquid inlet flow path 1312, the liquid outlet flow path 153 connected to the second heating container 1522 includes the first liquid outlet flow path 1531 and a third liquid outlet flow path 1533, the first liquid outlet flow path 1531 is connected to the blood return flow path 1305, and the third liquid outlet flow path 1533 is connected to the blood drawing flow path 1301 or the blood pumping flow path 1302.

Therefore, during actual usage, the replacement fluid in the second heating container 1522 may selectively flow into the blood return flow path 1305, the blood drawing flow path 1301 or the blood pumping flow path 1302.

Exemplarily, the first liquid outlet flow path 1531 may be controlled to be in communication and the third liquid outlet flow path 1533 may be controlled to open by a second pipeline switching unit 28 on the host 200, such that the replacement fluid in the replacement fluid supply container flows to the blood return flow path 1305 after passing through the fifth liquid inlet flow path 1311, the replacement fluid pump pipeline section 1125, the sixth liquid inlet flow path 1312, the second heating container 1522 and the first liquid outlet flow path 1531 sequentially, thereby achieving that the replacement fluid is injected into the blood filtered by the first filter element 300.

Alternatively, the first liquid outlet flow path 1531 may be controlled to open and the third liquid outlet flow path 1533 may be controlled to be in communication by the second pipeline switching unit 28 on the host 200, such that the replacement fluid in the replacement fluid supply container flows to the blood drawing flow path 1301 or the blood pumping flow path 1302 after passing through the fifth liquid inlet flow path 1311, the replacement fluid pump pipeline section 1125, the sixth liquid inlet flow path 1312, the second heating container 1522 and the third liquid outlet flow path 1533 sequentially, thereby achieving that the replacement fluid is injected into the blood that has not been filtered by the first filter element 300.

In some implementations, both the power panel 111 and the monitoring panel 121 are arranged separately from the heating panel 151, such that the heating panel 151 may be assembled and disassembled independently relative to the power panel 111, and the heating panel 151 may be assembled and disassembled independently relative to the monitoring panel 121.

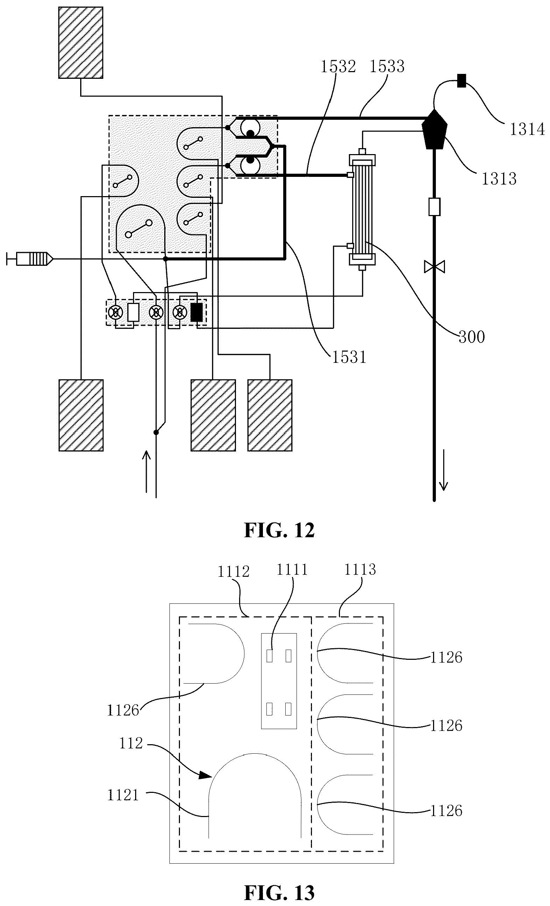

With continuous reference to FIG. 12, in some implementations, it is also possible that an output of the first liquid outlet flow path 1531 is connected to the blood drawing flow path 1301 or the blood pumping flow path 1302, an output of the second liquid outlet flow path 1532 is connected to the medical liquid input of the first filter element 300, and an output of the third liquid outlet flow path 1533 is connected to the blood return flow path 1305.

Therefore, the replacement fluid in the replacement fluid supply container may be selectively injected into the blood drawing flow path 1301 or the blood pumping flow path 1302 through the first liquid outlet flow path 1531, or injected into the blood return flow path 1305 through the third liquid outlet flow path 1533. Furthermore, the input of the third liquid inlet flow path 1309 may be selectively connected to the dialysate supply container and injected into the filter through the second liquid outlet flow path 1532, or the input of the third liquid inlet flow path 1309 may be selectively connected to the replacement fluid supply container and injected into the blood drawing flow path 1301 or the blood pumping flow path 1302 through the first liquid outlet flow path 1531.

In some implementations, it is also possible that the panel kit 100 is not provided with the heating panel 151 and the heating container 152. For example, the output of the fourth liquid inlet flow path 1310 is connected to an input of the first liquid outlet flow path 1531 and an input of the second liquid outlet flow path 1532 respectively through a three-way joint or the like, and the output of the sixth liquid inlet flow path 1312 is connected to the input of the first liquid outlet flow path 1531 and an input of the third liquid outlet flow path 1533 respectively through a three-way joint or the like.

At this time, the input of the first liquid outlet flow path 1531, the input of the second liquid outlet flow path 1532 and the input of the third liquid outlet flow path 1533 may be mounted to the power panel 111.

In some implementations, the blood return flow path 1305 is provided with a degassing pot 1313 and a degassing pot monitoring element 1314, the degassing pot monitoring element 1314 is communicated with the degassing pot 1313, and if the degassing pot monitoring element 1314 is connected to a degassing pot monitoring interface on the host 200, the degassing pot monitoring element 1314 is used for the blood purification device to monitor a pressure in the degassing pot 1313. For example, the degassing pot monitoring element 1314 may include a Luer connector.

With continuous reference to FIG. 13, in some implementations, each of the pump pipeline sections 112 is arc-shaped. Therefore, the pump pipeline section 112 may be wound around the pump head of the pump component 21 more conveniently through its arc-shaped structure.

In some implementations, openings formed at two ends of each of the pump pipeline sections 112 face toward an outer circumferential side of the power panel 111, therefore two ends of each of the pump pipeline sections 112 may be connected to other corresponding flow paths in the panel kit 100 conveniently by two ends of each of the pump pipeline sections 112 facing outward.

Optionally, openings formed at two ends of at least a portion of the pump pipeline sections 112 face an inner circumferential side of the power panel 111, which is not limited in the embodiment of the application.

In some implementations, the power panel 111 includes a first region 1112 and a second region 1113 adjacent to each other, and the first region 1112 is located at a side of the second region 1113 along a horizontal direction. The pump pipeline sections 112 includes one blood pump pipeline section 1121 and multiple medical liquid pump pipeline sections 1126, and if the blood pump pipeline section 1121 is adapted to the pump component 21, the blood pump pipeline section 1121 is configured to be driven by the pump component 21 so as to drive the blood to flow, and if the medical liquid pump pipeline sections 1126 are adapted to the pump components 21 respectively, the medical liquid pump pipeline sections 1126 are configured to be driven by the pump components 21 respectively to drive the medical liquid to flow. One of the medical liquid pump pipeline sections 1126 and the blood pump pipeline section 1121 are arranged substantially along a vertical direction and disposed on the first region 1112, and other medical liquid pump pipeline sections 1126 are arranged substantially along the vertical direction and disposed on the second region 1113. Therefore, with the pump pipeline sections 112 arranged in two rows, position on the power panel 111 may be fully utilized.

When it is mentioned that the first region 1112 is located at a side of the second region 1113 along the horizontal direction, it is possible that an upper end of the first region 1112 is flush with an upper end of the second region 1113, and a lower end of the first region 1112 is flush with a lower end of the second region 1113.

When it is mentioned that the first region 1112 is located at a side of the second region 1113 along the horizontal direction, it is also possible that the upper end of the first region 1112 is higher than the upper end of the second region 1113. At this time, the lower end of the first region 1112 may be lower than the lower end of the second region 1113, or the lower end of the first region 1112 may be higher than the lower end of the second region 1113, or the lower end of the first region 1112 may be flush with the lower end of the second region 1113.

When it is mentioned that the first region 1112 is located at a side of the second region 1113 along the horizontal direction, it is also possible that the upper end of the first region 1112 is lower than the upper end of the second region 1113. At this time, the lower end of the first region 1112 may be lower than the lower end of the second region 1113, or the lower end of the first region 1112 may be higher than the lower end of the second region 1113, or the lower end of the first region 1112 may be flush with the lower end of the second region 1113.

That is, in the embodiment of the application, when it is mentioned that the first region 1112 is located at a side of the second region 1113 along the horizontal direction, it is possible that the upper end of the first region 1112 is not flush with the upper end of the second region 1113, and the lower end of the first region 1112 is not flush with the lower end of the second region 1113.

In some embodiments, when it is mentioned that the first region 1112 is located at a side of the second region 1113 along the horizontal direction, it is possible that at least half of a region of the first region 1112 along the vertical direction is arranged directly opposite to the second region 1113 along the horizontal direction, and/or at least half of a region of the second region 1113 along the vertical direction is arranged directly opposite to the first region 1112 along the horizontal direction.

In some implementations, if the medical liquid pump pipeline sections 1126 are adapted to the pump components 21 respectively, one of the medical liquid pump pipeline section 1126 located on the first region 1112 and the medical liquid pump pipeline section 1126 located at a lowermost side of the second region 1113 is configured to be driven by the pump component 21 so as to drive the anticoagulant drug to flow, and the other one of the medical liquid pump pipeline section 1126 located on the first region 1112 and the medical liquid pump pipeline section 1126 located at a lowermost side of the second region 1113 is configured to be driven by the pump component 21 so as to drive the medical liquid flowing out of the first filter element 300 to flow. Alternatively, if the medical liquid pump pipeline sections 1126 are adapted to the pump components 21 respectively, one of the medical liquid pump pipeline section 1126 located on the first region 1112 and the medical liquid pump pipeline section 1126 located at a middle part of the second region 1113 is configured to be driven by the pump component 21 so as to drive the anticoagulant drug to flow, and the other one of the medical liquid pump pipeline section 1126 located on the first region 1112 and the medical liquid pump pipeline section 1126 located at a middle part of the second region 1113 is configured to be driven by the pump component 21 so as to drive the medical liquid flowing out of the first filter element 300 to flow.

For example, the medical liquid pump pipeline section 1126 configured to be driven by the pump component 21 so as to drive the anticoagulant drug to flow is the pump pipeline section of the front pump 1123, and the medical liquid pump pipeline section 1126 configured to be driven by the pump component 21 so as to drive the medical liquid flowing out of the first filter element 300 to flow is the waste liquid pressure pump pipeline section 1122.

Therefore, on one hand, no matter the medical liquid pump pipeline section 1126 located on the first region 1112 or the medical liquid pump pipeline section 1126 located at the lowermost side of the second region 1113 is the pump pipeline section of the front pump 1123, since the pump pipeline section of the front pump 1123 is adjacent to the blood pump pipeline section 1121, the blood drawing flow path 1301 or the blood pumping flow path 1302 connected to the blood pump pipeline section 1121 may be closer to the second liquid inlet flow path 1308 connected to the pump pipeline section of the front pump 1123, such that the second liquid inlet flow path 1308 may be shorter.

On the other hand, no matter the medical liquid pump pipeline section 1126 located on the first region 1112 or the medical liquid pump pipeline section 1126 located at the lowermost side of the second region 1113 is the waste liquid pressure pump pipeline section 1122, if the waste liquid pressure pump pipeline section 1122 is damaged, it is not easy for waste liquid of the medical liquid leaked from the waste liquid pressure pump pipeline section 1122 to contaminate other medical pump pipeline sections 112 located above the second region 1113.

In some implementations, the medical liquid pump pipeline sections 1126 may include at least four medical liquid pump pipeline sections. For example, the medical pump pipeline sections 112 includes the blood pump pipeline section 1121, the pump pipeline section of the front pump 1123, the dialysate pump pipeline section 1124 and the replacement fluid pump pipeline section 1125.

It may be understood that in the pump components 21 of the host 200, a pump body for pumping the blood may be larger than a pump body for pumping the medical liquid, to ensure that the pump body for pumping the blood may draw the blood out of the patient's blood vessel. Correspondingly, a radius of the blood pump pipeline section 1121 is also greater than that of the medical liquid pump pipeline section 1126. Therefore, with the blood pump pipeline section 1121 and one of the medical liquid pump pipeline sections 1126 arranged on the first region 1112, and at least other three of the medical liquid pump pipeline sections 1126 arranged on the second region 1113, space next to the first region 1112 may be fully utilized to integrate at least three medical liquid pump pipeline sections 1126, such that the structure of the power panel 111 may be more compact, such that structures of the power panel 111 are more compact, and corresponding structures of the pump components 21 on the host 200 may also be more compact.

Exemplarily, the pump pipeline sections 112 on the first region 1112 are the pump pipeline section of the front pump 1123 and the blood pump pipeline section 1121 arranged in sequence from top to bottom, and the pump pipeline sections 112 on the second region 1113 are the dialysate pump pipeline section 1124, the replacement fluid pump pipeline section 1125 and the waste liquid pressure pump pipeline section 1122 arranged in sequence from top to bottom.

Alternatively, the pump pipeline sections 112 on the first region 1112 are the pump pipeline section of the front pump 1123 and the blood pump pipeline section 1121 arranged in sequence from top to bottom, and the pump pipeline sections 112 on the second region 1113 are the dialysate pump pipeline section 1124, the waste liquid pressure pump pipeline section 1122 and the replacement fluid pump pipeline section 1125 arranged in sequence from top to bottom.

Alternatively, the pump pipeline sections 112 on the first region 1112 are the pump pipeline section of the front pump 1123 and the blood pump pipeline section 1121 arranged in sequence from top to bottom, and the pump pipeline sections 112 on the second region 1113 are the replacement fluid pump pipeline section 1125, the dialysate pump pipeline section 1124 and the waste liquid pressure pump pipeline section 1122 arranged in sequence from top to bottom.

Alternatively, the pump pipeline sections 112 on the first region 1112 are the pump pipeline section of the front pump 1123 and the blood pump pipeline section 1121 arranged in sequence from top to bottom, and the pump pipeline sections 112 on the second region 1113 are the replacement fluid pump pipeline section 1125, the waste liquid pressure pump pipeline section 1122 and the dialysate pump pipeline section 1124 arranged in sequence from top to bottom.

Alternatively, the pump pipeline sections 112 on the first region 1112 are the waste liquid pressure pump pipeline section 1122 and the blood pump pipeline section 1121 arranged in sequence from top to bottom, and the pump pipeline sections 112 on the second region 1113 are the dialysate pump pipeline section 1124, the replacement fluid pump pipeline section 1125 and the pump pipeline section of the front pump 1123 arranged in sequence from top to bottom.

Alternatively, the pump pipeline sections 112 on the first region 1112 are the waste liquid pressure pump pipeline section 1122 and the blood pump pipeline section 1121 arranged in sequence from top to bottom, and the pump pipeline sections 112 on the second region 1113 are the dialysate pump pipeline section 1124, the pump pipeline section of the front pump 1123 and the replacement fluid pump pipeline section 1125 arranged in sequence from top to bottom.

Alternatively, the pump pipeline sections 112 on the first region 1112 are the waste liquid pressure pump pipeline section 1122 and the blood pump pipeline section 1121 arranged in sequence from top to bottom, and the pump pipeline sections 112 on the second region 1113 are the replacement fluid pump pipeline section 1125, the dialysate pump pipeline section 1124 and the pump pipeline section of the front pump 1123 arranged in sequence from top to bottom.

Alternatively, the pump pipeline sections 112 on the first region 1112 are the waste liquid pressure pump pipeline section 1122 and the blood pump pipeline section 1121 arranged in sequence from top to bottom, and the pump pipeline sections 112 on the second region 1113 are the replacement fluid pump pipeline section 1125, the pump pipeline section of the front pump 1123 and the dialysate pump pipeline section 1124 arranged in sequence from top to bottom.

In some implementations, the first connection part 1111 of the power panel 111 may be located at least on the first region 1112. For example, at least a portion of the first connection part 1111 and the medical liquid pump pipeline section 1126 on the first region 1112 are arranged side by side along the horizontal direction and are located above the blood pump pipeline section 1121, therefore space above the blood pump pipeline section 1121 is fully utilized.

According to a second aspect, an embodiment of the application further provides a panel kit 100 for a blood purification device. The blood purification device may include a hemodialysis machine, a CRRT device or the like, which is not limited in the embodiment of the application. The blood purification device may further include a host 200. The host 200 is provided with pump components 21 and a retractable first interface 22.

The panel kit 100 may include a power component 11 and a monitoring component 12.

The power component 11 includes a power panel 111 and at least two pump pipeline sections 112. The power panel 111 is configured to be detachably mounted to the host 200, the at least two pump pipeline sections 112 are mounted to the power panel 111, at least one of the pump pipeline sections 112 is configured to cooperate with at least a corresponding one of the pump components 21 so as to drive a blood to flow along the at least one of the pump pipeline sections 112, and at least one of the pump pipeline sections 112 is configured to cooperate with at least corresponding one of the pump components 21 so as to drive a medical liquid to flow along the at least one of the pump pipeline sections 112. If the power panel 111 is mounted at a first target position of the host 200, the pump pipeline sections 112 are adapted to the pump components 21 respectively. If the pump pipeline sections 112 are adapted to the pump components 21 respectively, the pump pipeline sections 112 are capable of being driven by the pump components 21 respectively to drive the corresponding blood or medical liquid to flow. Therefore, after the power panel 111 is mounted at the first target position, it is unnecessary for the user to manually mount and fix the pump pipeline sections 112 to the pump components 21 respectively, such that installation of the power panel 111 is simpler, and a risk of mounting the at least two pump pipeline sections 112 at wrong positions may be reduced.

The monitoring component 12 includes a monitoring panel 121 and at least two monitoring pipeline sections 122. The monitoring panel 121 is configured to be detachably mounted to the host 200, the monitoring panel 121 is arranged separately from the power panel 111 such that the monitoring panel 121 and the power panel 111 can be assembled and disassembled separately, the at least two monitoring pipeline sections 122 are mounted to the monitoring panel 121, the at least two monitoring pipeline sections 122 are configured to monitor parameter information of the medical liquid and/or the blood, and the parameter information at least includes a pressure parameter; at least one of the monitoring pipeline sections 122 is provided with a second interface 1221. If the monitoring panel 121 is mounted at a second target position of the host 200, the first interface 22 can be extended to be connected to the second interface 1221, and if the second interface 1221 is connected to the first interface 22, the monitoring pipeline sections 122 are used for the host 200 to monitor the parameter information. Therefore, the user only needs to mount the monitoring panel 121 at the second target position of the host 200 simply, the first interfaces 22 on the host 200 may be extended to be connected to the second interfaces 1221 of the monitoring pipeline sections 122 respectively, and it is unnecessary for the user to mount the monitoring panel 121 at the second target position of the host 200 and then manually connect the first interfaces 22 to the second interfaces 1221 respectively, such that installation of the monitoring component 12 is simpler.

In some implementations, if the monitoring panel 121 is mounted at a second preset position of the host 200, the host 200 is capable of driving the monitoring panel 121 to move to a second target position. Therefore, for installation of the monitoring panel 121, the user only needs to mount the monitoring panel 121 at the second preset position of the host 201, and then the host 201 automatically drives the monitoring panel 121 to the second target position so as to achieve automatic installation of the monitoring panel 121, such that installation of the power component 11 is simpler.

In some implementations, if the monitoring panel 121 is mounted at the second preset position, the second interface 1221 is not connected to the first interface 22 on the host 200. Therefore, if the monitoring panel 121 is mounted at the second preset position, a certain gap may be reserved between the second interface 1221 and the first interface 22, such that the second interface 1221 may move toward the first interface 22 correspondingly when the monitoring panel 121 moves toward the second target position.

Furthermore, when the user needs to replace the panel kit 100 on the host 200 (that is, when the user needs to remove the panel kit 100 from the host 200), the host 200 may drive the monitoring panel 121 to move from the second target position to the second preset position so as to separate the second interface 1221 from the first interface 22, such that the user may remove the monitoring component 12 from the host 200 more conveniently.

In some implementations, whether the monitoring panel 121 is mounted at the second preset position and/or the second target position, is detectable by a second position detection mechanism 26 on the host 200.

Therefore, the host 200 may determine whether the monitoring panel 121 is mounted in place when the monitoring panel 121 is mounted at the second preset position, based on a detection result of the second position detection mechanism 26, thereby determining whether it needs to drive the monitoring panel 121 to move from the second preset position to the second target position; and after the host 200 drives the monitoring panel 121 to move from the second preset position to the second target position, the host 200 may further determine whether the monitoring panel 121 is mounted in place, based on the detection result of the second position detection mechanism 26.

In some implementations, the monitoring panel 121 is provided with a second connection part 1211, the second connection part 1211 is configured to be connectable to a second driving mechanism 25 of the host 200, such that the second driving mechanism 25 is capable of driving the monitoring panel 121 to move from the second preset position to the second target position.

For example, the second driving mechanism 25 is provided with a second claw 251 such that the second driving mechanism 25 may drive the second claw 251 to move, and specific structures of the second claw 251 and the second connection part 1211 may refer to specific structures of the second claw 251 and the second connection part 1211 according to the first aspect respectively, which are not elaborated here in the embodiment of the application.

Optionally, the second claw 251 may also be fixedly arranged on the host 200, and correspondingly, the blood purification device is not provided with the second driving mechanism 25, therefore the user may directly mount the monitoring panel 121 at the second target position, which is not limited in the embodiment of the application.

It may be understood that the panel kit 100 according to the second aspect differs from the panel kit 100 according to the first aspect in that: in the first aspect, the panel kit 100 may be manually mounted such that the monitoring panel 121 is mounted at the second target position of the host 200 and the second interface 1221 is synchronously connected to the first interface 22; however, in the second aspect, if the monitoring panel 121 is mounted at the second target position of the host 200, the first interface 22 on the host 200 may be extended to be connected to the second interface 1221.

Therefore, in some implementations, other specific structures of the panel kit 100 may refer to specific structures corresponding to the panel kit 100 according to the first aspect, which are not elaborated here in the embodiment of the application.

According to a third aspect, an embodiment of the application further provides a panel kit 100 for a blood purification device. The blood purification device may include a hemodialysis machine, a CRRT device or the like, which is not limited in the embodiment of the application. The blood purification device may further include a host 200. The host 200 is provided with pump components 21 and a first interface 22.

The panel kit 100 may include a power component 11 and a monitoring component 12.

The power component 11 includes a power panel 111 and at least two pump pipeline sections 112. The power panel 111 is configured to be detachably mounted to the host 200, the at least two pump pipeline sections 112 are mounted to the power panel 111, at least one of the pump pipeline sections 112 is configured to cooperate with at least a corresponding one of the pump components 21 so as to drive a blood to flow along the at least one of the pump pipeline sections 112, and at least one of the pump pipeline sections 112 is configured to cooperate with at least corresponding one of the pump components 21 so as to drive a medical liquid to flow along the at least one of the pump pipeline sections 112. If the power panel 111 is mounted at a first target position of the host 200, the pump pipeline sections 112 are adapted to the pump components 21 respectively. If the pump pipeline sections 112 are adapted to the pump components 21 respectively, the pump pipeline sections 112 are capable of being driven by the pump components 21 respectively to drive the corresponding blood or medical liquid to flow. Therefore, after the power panel 111 is mounted at the first target position, it is unnecessary for the user to manually mount and fix the pump pipeline sections 112 to the pump components 21 respectively, such that installation of the power panel 111 is simpler, and a risk of mounting the at least two pump pipeline sections 112 at wrong positions may be reduced.

The monitoring component 12 includes a monitoring panel 121 and at least two monitoring pipeline sections 122. The monitoring panel 121 is configured to be detachably mounted to the host 200, the monitoring panel 121 is arranged separately from the power panel 111 such that the monitoring panel 121 and the power panel 111 can be assembled and disassembled separately, the at least two monitoring pipeline sections 122 are mounted to the monitoring panel 121, the at least two monitoring pipeline sections 122 are configured to monitor parameter information of the medical liquid and/or the blood, and the parameter information at least includes a pressure parameter; at least one of the monitoring pipeline sections 122 is provided with a second interface 1221. If the monitoring panel 121 is mounted at a second preset position of the host 200, the host 200 is capable of driving the monitoring panel 121 to move to a second target position, such that the second interface 1221 is fixedly connected to the first interface 22. If the second interface 1221 is connected to the first interface 22, the monitoring pipeline sections 122 are used for the host 200 to monitor the parameter information. Therefore, for installation of the monitoring component 12, the user only needs to mount the monitoring panel 121 at the second preset position of the host 201, and then the host 201 automatically drives the monitoring panel 121 to the second target position so as to achieve automatic installation of the monitoring panel 121, such that installation of the power component 11 is simpler.

In some implementations, if the monitoring panel 121 is mounted at the second preset position, the second interface 1221 is not connected to the first interface 22 on the host 200. Therefore, if the monitoring panel 121 is mounted at the second preset position, a certain gap may be reserved between the second interface 1221 and the first interface 22, such that the second interface 1221 may move toward the first interface 22 correspondingly when the monitoring panel 121 moves toward the second target position.

Furthermore, when the user needs to replace the panel kit 100 on the host 200 (that is, when the user needs to remove the panel kit 100 from the host 200), the host 200 may drive the monitoring panel 121 to move from the second target position to the second preset position so as to separate the second interface 1221 from the first interface 22, such that the user may remove the monitoring component 12 from the host 200 more conveniently.

In some implementations, whether the monitoring panel 121 is mounted at the second preset position and/or the second target position, is detetable by a second position detection mechanism 26 on the host 200.

Therefore, the host 200 may determine whether the monitoring panel 121 is mounted in place when the monitoring panel 121 is mounted at the second preset position, based on a detection result of the second position detection mechanism 26, thereby determining whether it needs to drive the monitoring panel 121 to move from the second preset position to the second target position; and after the host 200 drives the monitoring panel 121 to move from the second preset position to the second target position, the host 200 may further determine whether the monitoring panel 121 is mounted in place, based on the detection result of the second position detection mechanism 26.

In some implementations, the monitoring panel 121 is provided with a second connection part 1211, the second connection part 1211 is configured to be connectable to a second driving mechanism 25 of the host 200, such that the second driving mechanism 25 is capable of driving the monitoring panel 121 to move from the second preset position to the second target position.

For example, the second driving mechanism 25 is provided with a second claw 251 such that the second driving mechanism 25 may drive the second claw 251 to move, and specific structures of the second claw 251 and the second connection part 1211 may refer to specific structures of the second claw 251 and the second connection part 1211 according to the first aspect respectively, which are not elaborated here in the embodiment of the application.

It may be understood that the panel kit 100 according to the third aspect differs from the panel kit 100 according to the first aspect in that: in the first aspect, the panel kit 100 may be manually mounted such that the monitoring panel 121 is mounted at the second target position of the host 200 and the second interface 1221 is synchronously connected to the first interface 22; however, in the third aspect, if the monitoring panel 121 is mounted at the second preset position of the host 200, the host 200 is capable of driving the monitoring panel 121 to move to the second target position, such that the second interface 1221 is connected to the first interface 22.

Therefore, in some implementations, other specific structures of the panel kit 100 may refer to specific structures of the panel kit 100 according to the first aspect, which are not elaborated here in the embodiment of the application.

According to a fourth aspect, an embodiment of the application further provides a panel kit 100 for a blood purification device. The blood purification device may include a hemodialysis machine, a CRRT device or the like, which is not limited in the embodiment of the application. The blood purification device may further include a host 200. The host 200 is provided with pump components 21 and a first interface 22.

The panel kit 100 may include a power component 11 and a monitoring component 12.

The power component 11 includes a power panel 111 and at least two pump pipeline sections 112. The power panel 111 is configured to be detachably mounted to the host 200, the at least two pump pipeline sections 112 are mounted to the power panel 111, at least one of the pump pipeline sections 112 is configured to cooperate with at least a corresponding one of the pump components 21 so as to drive a blood to flow along the at least one of the pump pipeline sections 112, and at least one of the pump pipeline sections 112 is configured to cooperate with at least corresponding one of the pump components 21 so as to drive a medical liquid to flow along the at least one of the pump pipeline sections 112. If the power panel 111 is mounted at a first target position of the host 200, the pump pipeline sections 112 are adapted to the pump components 21 respectively. If the pump pipeline sections 112 are adapted to the pump components 21 respectively, the pump pipeline sections 112 are capable of being driven by the pump components 21 respectively to drive the corresponding blood or medical liquid to flow. Therefore, after the power panel 111 is mounted at the first target position, it is unnecessary for the user to manually mount and fix the pump pipeline sections 112 to the pump components 21 respectively, such that installation of the power panel 111 is simpler, and a risk of mounting the at least two pump pipeline sections 112 at wrong positions may be reduced.

The monitoring component 12 includes a monitoring panel 121 and at least two monitoring pipeline sections 122. The monitoring panel 121 is configured to be detachably mounted to the host 200, the monitoring panel 121 is arranged separately from the power panel 111 such that the monitoring panel 121 and the power panel 111 are independent of each other and can be assembled and disassembled separately. The at least two monitoring pipeline sections 122 are mounted to the monitoring panel 121, the at least two monitoring pipeline sections 122 are configured to monitor parameter information of the medical liquid and/or the blood, and the parameter information at least includes a pressure parameter. At least one of the monitoring pipeline sections 122 is provided with a second interface 1221. The second interface 1221 is movably arranged relative to the monitoring panel 121, and if the monitoring panel 121 is mounted at a second target position of the host 200, the second interface 1221 is capable of being manually operated to achieve connection of the first interface 22 to the second interface 1221. If the second interface 1221 is connected to the first interface 22, the monitoring pipeline sections 122 are used for the host 200 to monitor the parameter information.

After the monitoring panel 121 is mounted in place, it may facilitate the user to manually connect the first interface 22 to the second interface 1221, to avoid failure to align and seal between the first interface 22 and the second interface 1221.

In an embodiment, in actual production and manufacturing processes, it is inevitable that each part has a form tolerance, and it is also inevitable that each part has a position tolerance there-between during assembly of each part. Therefore, after the monitoring panel 121 is mounted at the second target position, it is enough for the user to manually operate to achieve connection of the first interface 22 to the second interface 1221, then it is unnecessary to use a manufacturing process with high cost and great difficulty to ensure that an accumulated geometric tolerance of each part such as the first interface 22, the monitoring panel 121, the blood purification device or the like is located in an acceptable range so as to achieve that the first interface 22 and the monitoring panel 121 are mounted in place once, and difficulty of manufacturing the monitoring component 12 and difficulty of mounting the monitoring component 12 to the device housing 201 may be reduced finally.

Furthermore, in an actual assembly process of mounting the panel kit 100 to the blood purification device, when there is a large number of parts required to be mounted in place once, such as the monitoring panel 121, the first interface 22 or the like, it is difficult to ensure that all these parts may be mounted in place synchronously. Therefore, after the monitoring panel 121 is mounted in place, the user manually connects the first interface 22 to the second interface 1221, which may make installation of the panel kit 100 simpler and more convenient.

In an embodiment, connection of the first interface 22 to the second interface 1221 may be sealed connection between the first interface 22 and the second interface 1221.

In some implementations, the first interface 22 may be connected to the second interface 1221 by screws, snap-fit, plug-in, etc. Based on this, if the monitoring panel 121 is mounted at the second target position, the first interface 22 may be manually operated by tightening the first interface 22, pressing the first interface 22, plugging the first interface 22 or the like, to achieve connection of the first interface 22 to the second interface 1221, which is not limited in the embodiment of the application.

For example, the first interface 22 is provided with a knob. If the monitoring panel 121 is mounted at the second target position, connection of the first interface 22 to the second interface 1221 may be achieved by an operation of turning the knob of the first interface 22 by the user.

In some implementations, it is also possible that the user directly mounts the monitoring panel 121 at the second target position. Therefore, the panel kit 100 according to the fourth aspect differs from the panel kit 100 according to the first aspect in that:

in the first aspect, the panel kit 100 may be manually mounted such that the monitoring panel 121 is mounted at the second target position of the host 200 and the second interface 1221 is synchronously connected to the first interface 22; however, in the fourth aspect, if the monitoring panel 121 is mounted at the second preset position of the host 200, the host 200 is capable of driving the monitoring panel 121 to move to the second target position, and then connection of the first interface 22 to the second interface 1221 is achieved by the user's manual operation.

Therefore, in some implementations, other specific structures of the panel kit 100 may refer to specific structures of the panel kit 100 according to the first aspect, which are not elaborated here in the embodiment of the application.

Optionally, it is also possible that the user manually mounts the monitoring panel 121 at the second preset position, and then the blood purification device automatically drives the monitoring panel 121 to the second target position. Therefore, the panel kit 100 according to the fourth aspect differs from the panel kit 100 according to the third aspect in that:

in the third aspect, if the monitoring panel 121 is mounted at the second preset position of the host 200, the host 200 is capable of driving the monitoring panel 121 to move to the second target position, such that the second interface 1221 is connected to the first interface 22; however, in the fourth aspect, if the monitoring panel 121 is mounted at the second preset position of the host 200, the host 200 is capable of driving the monitoring panel 121 to move to the second target position, and then connection of the first interface 22 to the second interface 1221 is achieved by the user's manual operation.

Therefore, in some implementations, other specific structures of the panel kit 100 may refer to specific structures of the panel kit 100 according to the third aspect, which are not elaborated here in the embodiment of the application.

According to a fifth aspect, an embodiment of the application further provides a panel kit 100 for a blood purification device. The blood purification device may include a hemodialysis machine, a CRRT device or the like, which is not limited in the embodiment of the application. The blood purification device includes a host 200, the host 200 is provided with pump components 21, a first interface 22 and a conductive member.

The panel kit 100 includes a power component 11 and a monitoring component 12.

The power component 11 includes a power panel 111 and at least two pump pipeline sections 112. The power panel 111 is configured to be detachably mounted to the host 200, the at least two pump pipeline sections 112 are mounted to the power panel 111, at least one of the pump pipeline sections 112 is configured to cooperate with at least a corresponding one of the pump components 21 so as to drive a blood to flow along the at least one of the pump pipeline sections 112, and at least one of the pump pipeline sections 112 is configured to cooperate with at least corresponding one of the pump components 21 so as to drive a medical liquid to flow along the at least one of the pump pipeline sections 112. If the power panel 111 is mounted at a first target position of the host 200, the pump pipeline sections 112 are adapted to the pump components 21 respectively. If the pump pipeline sections 112 are adapted to the pump components 21 respectively, the pump pipeline sections 112 are capable of being driven by the pump components 21 respectively to drive the corresponding blood or medical liquid to flow. Therefore, after the power panel 111 is mounted at the first target position, it is unnecessary for the user to manually mount and fix the pump pipeline sections 112 to the pump components 21 respectively, such that installation of the power panel 111 is simpler, and a risk of mounting the at least two pump pipeline sections 112 at wrong positions may be reduced.

The monitoring component 12 includes a monitoring panel 121, monitoring pipeline sections 122 and an ESD element 14. The monitoring panel 121 is configured to be detachably mounted to the host 200, the monitoring panel 121 is arranged separately from the power panel 111 such that the monitoring panel 121 and the power panel 111 can be assembled and disassembled separately. Each of the monitoring pipeline sections 122 and the ESD element 14 are mounted to the monitoring panel 121, the monitoring pipeline sections 122 are configured to monitor parameter information of the medical liquid and/or the blood, and the parameter information at least includes a pressure parameter. At least one of the monitoring pipeline sections 122 is provided with a second interface 1221; the second interface 1221 is fixedly arranged on the monitoring panel 121, the monitoring component 12 is capable of being manually mounted such that the monitoring panel 121 is mounted at a second target position of the host 200, the second interface 1221 is synchronously connected to the first interface 22 and the ESD element 14 is synchronously connected to the conductive member. If the second interface 1221 is connected to the first interface 22, the monitoring pipeline sections 122 are used for the host 200 to monitor the parameter information. If the ESD element 14 is connected to the conductive member, the ESD element 14 is capable of discharging static electricity from the panel kit 100. Therefore, operations of mounting the second interface 1221 and the ESD element 14 independently may be reduced, and accuracy of mounting the second interface 1221 and the ESD element 14 may be improved. As may be seen, the panel kit 100 according to the embodiment of the application may reduce difficulty of mounting pipelines in the panel kit 100 and reduce a probability of mounting the pipelines in the panel kit 100 at wrong positions.

Here, it should be noted that when it is mentioned that the monitoring component 12 is capable of being manually mounted such that the monitoring panel 121 is mounted at a second target position of the host 200, the second interface 1221 is synchronously connected to the first interface 22 and the ESD element 14 is synchronously connected to the conductive member, it may be understood that the operation of mounting the monitoring panel 121 at the second target position may drive the second interface 1221 to be connected to the first interface 22 and drive the ESD element 14 to be connected to the conductive member, thereby achieving that installation and fixation of two parts, that is, the monitoring panel 121, the second interface 1221 and the ESD element 14 are completed by one action. In this process, the operation of mounting the monitoring panel 121 at the second target position, the operation of connecting the second interface 1221 to the first interface 22 and the operation of connecting the ESD element 14 to the conductive member may be completed simultaneously, or there may be a certain time difference there-between, which is not limited in the embodiment of the application.

It may be understood that the panel kit 100 according to the fifth aspect differs from the panel kit 100 according to the first aspect in that: in the first aspect, the monitoring panel 121 is provided with at least two monitoring pipeline sections 122; however, in the fifth aspect, the monitoring panel 121 may be provided with only the ESD element 14 and one monitoring pipeline section 122, and of course, it is also possible that the monitoring panel 121 may be provided with only the ESD element 14 and multiple monitoring pipeline sections 122, which is not limited in the embodiment of the application.

Therefore, in some implementations, other specific structures of the panel kit 100 may refer to specific structures of the panel kit 100 according to the first aspect, which are not elaborated here in the embodiment of the application.

According to a sixth aspect, an embodiment of the application further provides a panel kit 100 for a blood purification device. The blood purification device may include a hemodialysis machine, a CRRT device or the like, which is not limited in the embodiment of the application. The blood purification device includes a host 200, the host 200 is provided with pump components 21, a first interface 22 and a conductive member.

The panel kit 100 includes a power component 11 and a monitoring component 12.

The power component 11 includes a power panel 111 and at least two pump pipeline sections 112. The power panel 111 is configured to be detachably mounted to the host 200, the at least two pump pipeline sections 112 are mounted to the power panel 111, at least one of the pump pipeline sections 112 is configured to cooperate with at least a corresponding one of the pump components 21 so as to drive a blood to flow along the at least one of the pump pipeline sections 112, and at least one of the pump pipeline sections 112 is configured to cooperate with at least corresponding one of the pump components 21 so as to drive a medical liquid to flow along the at least one of the pump pipeline sections 112. If the power panel 111 is mounted at a first target position of the host 200, the pump pipeline sections 112 are adapted to the pump components 21 respectively. If the pump pipeline sections 112 are adapted to the pump components 21 respectively, the pump pipeline sections 112 are capable of being driven by the pump components 21 respectively to drive the corresponding blood or medical liquid to flow. Therefore, after the power panel 111 is mounted at the first target position, it is unnecessary for the user to manually mount and fix the pump pipeline sections 112 to the pump components 21 respectively, such that installation of the power panel 111 is simpler, and a risk of mounting the at least two pump pipeline sections 112 at wrong positions may be reduced.

The monitoring component 12 includes a monitoring panel 121, monitoring pipeline sections 122 and an ESD element 14. The monitoring panel 121 is configured to be detachably mounted to the host 200, the monitoring panel 121 is arranged separately from the power panel 111 such that the monitoring panel 121 and the power panel 111 can be assembled and disassembled separately. Each of the monitoring pipeline sections 122 and the ESD element 14 are mounted to the monitoring panel 121, the monitoring pipeline sections 122 are configured to monitor parameter information of the medical liquid and/or the blood, and the parameter information at least includes a pressure parameter. At least one of the monitoring pipeline sections 122 is provided with a second interface 1221. The second interface 1221 is fixedly arranged on the monitoring panel 121, and if the monitoring panel 121 is mounted at a second target position of the host 200, the first interface 22 can be extended to be connected to the second interface 1221 fixedly, the ESD element 14 is connected to the conductive member fixedly arranged on the host 200, or the ESD element 14 is connected to the conductive member extended toward an exterior of the host 200. If the second interface 1221 is connected to the first interface 22, the monitoring pipeline sections 122 are used for the host 200 to monitor the parameter information. If the ESD element 14 is connected to the conductive member, the ESD element 14 is capable of discharging static electricity from the panel kit 100. Therefore, operations of mounting the second interface 1221 and the ESD element 14 independently may be reduced, and accuracy of mounting the second interface 1221 and the ESD element 14 may be improved. As may be seen, the panel kit 100 according to the embodiment of the application may reduce difficulty of mounting pipelines in the panel kit 100 and reduce a probability of mounting the pipelines in the panel kit 100 at wrong positions.

It may be understood that the panel kit 100 according to the sixth aspect differs from the panel kit 100 according to the second aspect in that: in the second aspect, the monitoring panel 121 is provided with at least two monitoring pipeline sections 122; however, in the sixth aspect, the monitoring panel 121 may be provided with only the ESD element 14 and one monitoring pipeline section 122, and of course, it is also possible that the monitoring panel 121 may be provided with only the ESD element 14 and multiple monitoring pipeline sections 122, which is not limited in the embodiment of the application.

Therefore, in some implementations, other specific structures of the panel kit 100 may refer to specific structures of the panel kit 100 according to the second aspect, which are not elaborated here in the embodiment of the application.

According to a seventh aspect, an embodiment of the application further provides a panel kit 100 for a blood purification device. The blood purification device may include a hemodialysis machine, a CRRT device or the like, which is not limited in the embodiment of the application. The blood purification device includes a host 200, the host 200 is provided with pump components 21, a first interface 22 and a conductive member.

The panel kit 100 includes a power component 11 and a monitoring component 12.

The power component 11 includes a power panel 111 and at least two pump pipeline sections 112. The power panel 111 is configured to be detachably mounted to the host 200, the at least two pump pipeline sections 112 are mounted to the power panel 111, at least one of the pump pipeline sections 112 is configured to cooperate with at least a corresponding one of the pump components 21 so as to drive a blood to flow along the at least one of the pump pipeline sections 112, and at least one of the pump pipeline sections 112 is configured to cooperate with at least corresponding one of the pump components 21 so as to drive a medical liquid to flow along the at least one of the pump pipeline sections 112. If the power panel 111 is mounted at a first target position of the host 200, the pump pipeline sections 112 are adapted to the pump components 21 respectively. If the pump pipeline sections 112 are adapted to the pump components 21 respectively, the pump pipeline sections 112 are capable of being driven by the pump components 21 respectively to drive the corresponding blood or medical liquid to flow. Therefore, after the power panel 111 is mounted at the first target position, it is unnecessary for the user to manually mount and fix the pump pipeline sections 112 to the pump components 21 respectively, such that installation of the power panel 111 is simpler, and a risk of mounting the at least two pump pipeline sections 112 at wrong positions may be reduced.

The monitoring component 12 includes a monitoring panel 121, monitoring pipeline sections 122 and an ESD element 14. The monitoring panel 121 is configured to be detachably mounted to the host 200, the monitoring panel 121 is arranged separately from the power panel 111 such that the monitoring panel 121 and the power panel 111 can be assembled and disassembled separately. Each of the monitoring pipeline sections 122 and the ESD element 14 are mounted to the monitoring panel 121, the monitoring pipeline sections 122 are configured to monitor parameter information of the medical liquid and/or the blood, and the parameter information at least includes a pressure parameter; at least one of the monitoring pipeline sections 122 is provided with a second interface 1221; the second interface 1221 is fixedly arranged on the monitoring panel 121, and if the monitoring panel 121 is mounted at a second preset position of the host 200, the host 200 is capable of driving the monitoring panel 121 to move to a second target position, such that the second interface 1221 is fixedly connected to the first interface 22 and the ESD element 14 is connected to the conductive member. If the second interface 1221 is connected to the first interface 22, the monitoring pipeline sections 122 are used for the host 200 to monitor the parameter information. If the ESD element 14 is connected to the conductive member, the ESD element 14 is capable of discharging static electricity from the panel kit 100. Therefore, operations of mounting the second interface 1221 and the ESD element 14 independently may be reduced, and accuracy of mounting the second interface 1221 and the ESD element 14 may be improved. As may be seen, the panel kit 100 according to the embodiment of the application may reduce difficulty of mounting pipelines in the panel kit 100 and reduce a probability of mounting the pipelines in the panel kit 100 at wrong positions.

It may be understood that the panel kit 100 according to the seventh aspect differs from the panel kit 100 according to the third aspect in that: in the third aspect, the monitoring panel 121 is provided with at least two monitoring pipeline sections 122; however, in the seventh aspect, the monitoring panel 121 may be provided with only the ESD element 14 and one monitoring pipeline section 122, and of course, it is also possible that the monitoring panel 121 may be provided with only the ESD element 14 and multiple monitoring pipeline sections 122, which is not limited in the embodiment of the application.

Therefore, in some implementations, other specific structures of the panel kit 100 may refer to specific structures of the panel kit 100 according to the third aspect, which are not elaborated here in the embodiment of the application.

According to an eighth aspect, an embodiment of the application further provides a panel kit 100 for a blood purification device. The blood purification device may include a hemodialysis machine, a CRRT device or the like, which is not limited in the embodiment of the application. The blood purification device includes a host 200, the host 200 is provided with pump components 21, a first interface 22 and a conductive member.

The panel kit 100 may include a power component 11 and a monitoring component 12.

The power component 11 includes a power panel 111 and at least two pump pipeline sections 112. The power panel 111 is configured to be detachably mounted to the host 200, the at least two pump pipeline sections 112 are mounted to the power panel 111, at least one of the pump pipeline sections 112 is configured to cooperate with at least a corresponding one of the pump components 21 so as to drive a blood to flow along the at least one of the pump pipeline sections 112, and at least one of the pump pipeline sections 112 is configured to cooperate with at least corresponding one of the pump components 21 so as to drive a medical liquid to flow along the at least one of the pump pipeline sections 112. If the power panel 111 is mounted at a first target position of the host 200, the pump pipeline sections 112 are adapted to the pump components 21 respectively. If the pump pipeline sections 112 are adapted to the pump components 21 respectively, the pump pipeline sections 112 are capable of being driven by the pump components 21 respectively to drive the corresponding blood or medical liquid to flow. Therefore, after the power panel 111 is mounted at the first target position, it is unnecessary for the user to manually mount and fix the pump pipeline sections 112 to the pump components 21 respectively, such that installation of the power panel 111 is simpler, and a risk of mounting the at least two pump pipeline sections 112 at wrong positions may be reduced.

The monitoring component 12 includes a monitoring panel 121, monitoring pipeline sections 122 and an ESD element 14. The monitoring panel 121 is configured to be detachably mounted to the host 200. The monitoring panel 121 is arranged separately from the power panel 111 such that the monitoring panel 121 and the power panel 111 are independent of each other and can be assembled and disassembled separately. Each of the monitoring pipeline sections 122 and the ESD element 14 are mounted to the monitoring panel 121. The monitoring pipeline sections 122 are configured to monitor parameter information of the medical liquid and/or the blood, and the parameter information at least includes a pressure parameter. At least one of the monitoring pipeline sections 122 is provided with a second interface 1221. The second interface 1221 is movably arranged relative to the monitoring panel 121, and if the monitoring panel 121 is mounted at a second target position of the host 200, the second interface 1221 is capable of being manually operated to achieve connection of the first interface 22 to the second interface 1221. If the second interface 1221 is connected to the first interface 22, the monitoring pipeline sections 122 are used for the host 200 to monitor the parameter information. If the ESD element 14 is connected to the conductive member, the ESD element 14 is capable of discharging static electricity from the panel kit 100.

Therefore, after the monitoring panel 121 is mounted in place, it may facilitate the user to manually connect the first interface 22 to the second interface 1221, to avoid failure to align and seal between the first interface 22 and the second interface 1221. Furthermore, with the ESD element 14 integrated into the monitoring panel 121, it may also facilitate installation of the ESD element 14.

It may be understood that the panel kit 100 according to the eighth aspect differs from the panel kit 100 according to the fourth aspect in that: in the fourth aspect, the monitoring panel 121 is provided with at least two monitoring pipeline sections 122; however, in the eighth aspect, the monitoring panel 121 may be provided with only the ESD element 14 and one monitoring pipeline section 122, and of course, it is also possible that the monitoring panel 121 may be provided with only the ESD element 14 and multiple monitoring pipeline sections 122, which is not limited in the embodiment of the application.

Therefore, in some implementations, other specific structures of the panel kit 100 may refer to specific structures of the panel kit 100 according to the fourth aspect, which are not elaborated here in the embodiment of the application.

In the above embodiments, description of each of the embodiments has its own emphasis, and portions that are not described in detail in a certain embodiment may refer to relevant descriptions of other embodiments.

The panel kit 100 for a blood purification device provided in the embodiments of the application have been described in detail as above, principles and implementations of the application have been described here by using specific examples, and descriptions of the above embodiments are only intended to help to understand methods and core ideas of the application; furthermore, variations of specific implementations and scope of the application may be made by those skilled in the art according to the ideas of the application, and in summary, contents of the description should not be understood as limiting the application.

The following is a list of enumerated example embodiments.
1. A panel kit for a blood purification device, the blood purification device comprises a host provided with pump components and a first interface, and the panel kit comprises:
   a power component, comprising a power panel and at least two pump pipeline sections, wherein the power panel is configured to be detachably mounted to the host, the at least two pump pipeline sections are mounted to the power panel, at least one of the pump pipeline sections is configured to cooperate with at least a corresponding one of the pump components so as to drive a blood to flow along the at least one of the pump pipeline sections, and at least one of the pump pipeline sections is configured to cooperate with at least another one of the pump components so as to drive a medical liquid to flow along the at least one of the pump pipeline sections; if the power panel is mounted at a first target position of the host, the pump pipeline sections are adapted to the pump components respectively, and if the pump pipeline sections are adapted to the pump components respectively, the pump pipeline sections are capable of being driven by the pump components respectively to drive the corresponding blood or medical liquid to flow; and
   a monitoring component, comprising a monitoring panel and at least two monitoring pipeline sections, wherein the monitoring panel is configured to be detachably mounted to the host, the monitoring panel is arranged separately from the power panel such that the monitoring panel and the power panel are independent of each other and can be assembled and disassembled separately, the at least two monitoring pipeline sections are mounted to the monitoring panel and are configured to monitor parameter information of the medical liquid and/or the blood, and the parameter information at least comprises a pressure parameter; at least one of the monitoring pipeline sections is provided with a second interface; the monitoring component is capable of being manually mounted such that the monitoring panel is mounted at a second target position of the host and the second interface is synchronously connected to the first interface, and if the second interface is connected to the first interface, the monitoring pipeline sections are used for the host to monitor the parameter information.
2. A panel kit for a blood purification device, the blood purification device comprises a host provided with pump components and a retractable first interface, and the panel kit comprises:
   a power component, comprising a power panel and at least two pump pipeline sections, wherein the power panel is configured to be detachably mounted to the host, the at least two pump pipeline sections are mounted to the power panel, at least one of the pump pipeline sections is configured to cooperate with at least a corresponding one of the pump components so as to drive a blood to flow along the at least one of the pump pipeline sections, and at least one of the pump pipeline sections is configured to cooperate with at least corresponding one of the pump components so as to drive a medical liquid to flow along the at least one of the pump pipeline sections; if the power panel is mounted at a first target position of the host, the pump pipeline sections are adapted to the pump components respectively, and if the pump pipeline sections are adapted to the pump components respectively, the pump pipeline sections are capable of being driven by the pump components respectively to drive the corresponding blood or medical liquid to flow; and
   a monitoring component, comprising a monitoring panel and at least two monitoring pipeline sections, wherein the monitoring panel is configured to be detachably mounted to the host, the monitoring panel is arranged separately from the power panel such that the monitoring panel and the power panel are independent of each other and can be assembled and disassembled separately, the at least two monitoring pipeline sections are mounted to the monitoring panel and are configured to monitor parameter information of the medical liquid and/or the blood, and the parameter information at least comprises a pressure parameter; at least one of the monitoring pipeline sections is provided with a second interface, and if the monitoring panel is mounted at a second target position of the host, the first interface can be extended to be connected to the second interface, and if the second interface is connected to the first interface, the monitoring pipeline sections are used for the host to monitor the parameter information.
3. A panel kit for a blood purification device, the blood purification device comprises a host provided with pump components and a first interface, and the panel kit comprises:
   a power component, comprising a power panel and at least two pump pipeline sections, wherein the power panel is configured to be detachably mounted to the host, the at least two pump pipeline sections are mounted to the power panel, at least one of the pump pipeline sections is configured to cooperate with at least a corresponding one of the pump components so as to drive a blood to flow along the at least one of the pump pipeline sections, and at least one of the pump pipeline sections is configured to cooperate with at least corresponding one of the pump components so as to drive a medical liquid to flow along the at least one of the pump pipeline sections; if the power panel is mounted at a first target position of the host, the pump pipeline sections are adapted to the pump components respectively, and if the pump pipeline sections are adapted to the pump components respectively, the pump pipeline sections are capable of being driven by the pump components respectively to drive the corresponding blood or medical liquid to flow; and
   a monitoring component, comprising a monitoring panel and at least two monitoring pipeline sections, wherein the monitoring panel is configured to be detachably mounted to the host, the monitoring panel is arranged separately from the power panel such that the monitoring panel and the power panel are independent of each other and can be assembled and disassembled separately, the at least two monitoring pipeline sections are mounted to the monitoring panel and are configured to monitor parameter information of the medical liquid and/or the blood, and the parameter information at least comprises a pressure parameter; at least one of the monitoring pipeline sections is provided with a second interface; if the monitoring panel is mounted at a second preset position of the host, the host is capable of driving the monitoring panel to move to a second target position, such that the second interface is connected to the first interface, and if the second interface is connected to the first interface, the monitoring pipeline sections are used for the host to monitor the parameter information.
4. A panel kit for a blood purification device, the blood purification device comprises a host provided with pump components and a first interface, and the panel kit comprises:
   a power component, comprising a power panel and at least two pump pipeline sections, wherein the power panel is configured to be detachably mounted to the host, the at least two pump pipeline sections are mounted to the power panel, at least one of the pump pipeline sections is configured to cooperate with at least a corresponding one of the pump components so as to drive a blood to flow along the at least one of the pump pipeline sections, and at least one of the pump pipeline sections is configured to cooperate with at least corresponding one of the pump components so as to drive a medical liquid to flow along the at least one of the pump pipeline sections; if the power panel is mounted at a first target position of the host, the pump pipeline sections are adapted to the pump components respectively, and if the pump pipeline sections are adapted to the pump components respectively, the pump pipeline sections are capable of being driven by the pump components respectively to drive the corresponding blood or medical liquid to flow; and
   a monitoring component, comprising a monitoring panel and at least two monitoring pipeline sections, wherein the monitoring panel is configured to be detachably mounted to the host, the monitoring panel is arranged separately from the power panel such that the monitoring panel and the power panel are independent of each other and can be assembled and disassembled separately, the at least two monitoring pipeline sections are mounted to the monitoring panel and are configured to monitor parameter information of the medical liquid and/or the blood, and the parameter information at least comprises a pressure parameter; at least one of the monitoring pipeline sections is provided with a second interface; the second interface is movably arranged relative to the monitoring panel, and if the monitoring panel is mounted at a second target position of the host, the second interface is capable of being manually operated to achieve connection of the first interface to the second interface, and if the second interface is connected to the first interface, the monitoring pipeline sections are used for the host to monitor the parameter information.
5. The panel kit of any one of examples 1 to 3, wherein the second interface is fixedly arranged relative to the monitoring panel; or
   the second interface is movably arranged relative to the monitoring panel.
6. The panel kit of any one of examples 1 to 4, wherein the pump pipeline sections comprise a first flexible pipeline section abutting against a pump head of one of the pump components if the pump pipeline sections are adapted to the pump components respectively, such that rotation of the pump head is capable of driving the first flexible pipeline to be wound around the pump head.
7. The panel kit of any one of examples 1 to 4, wherein if the power panel is mounted at a first preset position of the host, the power panel is capable of being driven by the host to move to the first target position, such that the pump pipeline sections are adapted to the pump components respectively.
8. The panel kit of example 7, wherein if the power panel is mounted at the first preset position, the pump pipeline sections are not adapted to the pump components respectively; and/or
   whether the power panel is mounted at the first preset position and/or the first target position, is detectable by a first position detection mechanism on the host; and/or
   the power panel is provided with a first connection part configured to be connectable to a first driving mechanism of the host, such that the first driving mechanism is capable of driving the power panel to move from the first preset position to the first target position.
9. The panel kit of any one of examples 1 to 4, wherein the power component is capable of being manually mounted such that the power panel is mounted at the first target position and the pump pipeline sections are synchronously adapted to the pump components respectively.
10. The panel kit of example 9, wherein whether the power panel is mounted at the first target position, is detectable by a first position detection mechanism on the host.
11. The panel kit of any one of examples 1, 2 and 4, wherein whether the monitoring panel is mounted at the second target position, is detectable by a second position detection mechanism on the host.
12. The panel kit of example 3, wherein if the monitoring panel is mounted at the second preset position, the second interface is not connected to the first interface; and/or
   whether the monitoring panel is mounted at the second preset position and/or the second target position, is detectable by a second position detection mechanism on the host; and/or
   the monitoring panel is provided with a second connection part configured to be connectable to a second driving mechanism of the host, such that the second driving mechanism is capable of driving the monitoring panel to move from the second preset position to the second target position.
13. The panel kit of any one of examples 1 to 4, further comprising a second flexible pipeline section connected between at least one of the pump pipeline sections and at least one of the monitoring pipeline sections.
14. The panel kit of any one of examples 1 to 4, wherein at least two of the monitoring pipeline sections are provided with the second interfaces respectively, and the first interface comprises at least two first interfaces, and the second interfaces are configured to be connected to the corresponding first interfaces on the host respectively, such that the host is capable of monitoring the pressure parameter of the corresponding blood or medical liquid through the monitoring pipeline sections; and/or
   the blood purification device further comprises a first filter element configured to filter the blood flowing through the pump pipeline sections; if the second interfaces are connected to the first interfaces respectively, the monitoring pipeline sections are used for the host to monitor the pressure parameter of the corresponding blood or medical liquid; the at least two monitoring pipeline sections further comprise a blood leakage monitoring pipeline section configured to cooperate with a blood leakage monitoring module on the host, so as to monitor whether the blood at one side of a filtration membrane of the first filter element permeates to the other side of the filtration membrane.
15. The panel kit of any one of examples 1 to 4, wherein the blood purification device further comprises a first filter element configured to filter the blood flowing through the pump pipeline sections;
   the monitoring pipeline sections comprise at least one of a blood leakage monitoring pipeline section, a blood drawing pressure monitoring pipeline section, a pressure monitoring pipeline section before filtering and a waste liquid pressure monitoring pipeline section;
   the blood leakage monitoring pipeline section is configured to cooperate with a blood leakage monitoring module on the host, so as to monitor whether the blood at one side of a filtration membrane of the first filter element permeates to the other side of the filtration membrane;
   the blood drawing pressure monitoring pipeline section is configured to monitor the pressure parameter of the blood drawn out of a patient's blood vessel;
   the pressure monitoring pipeline section before filtering is configured to monitor a pressure parameter of the blood flowing from the pump pipeline sections into the first filter element;
   the waste liquid pressure monitoring pipeline section is configured to monitor a pressure parameter of the medical liquid discharged from the first filter element.
16. The panel kit of example 15, further comprising an electrostatic discharge (ESD) element arranged on at least one of the power panel and the monitoring panel and configured to cooperate with the host so as to discharge static electricity from the panel kit.
17. The panel kit of example 15, wherein the blood purification device further comprises a second filter element configured to re-filter the blood flowing through the pump pipeline sections, a blood input of the second filter element is fluidly communicated with a blood output of the first filter element so as to re-filter the blood flowing out of the blood output of the first filter element;
   the monitoring pipeline sections further comprise a secondary membrane pressure monitoring pipeline section configured to fluidly communicate the first filter element with the second filter element and to monitor the pressure parameter of the blood.
18. The panel kit of example 17, wherein the monitoring component comprises at least two monitoring components, any two of the monitoring components are arranged separately from each other, such that each of the monitoring components can be assembled and disassembled separately, and each of the monitoring components is provided with at least two of the blood leakage monitoring pipeline section, the blood drawing pressure monitoring pipeline section, the pressure monitoring pipeline section before filtering and the waste liquid pressure monitoring pipeline section.
19. The panel kit of example 15, wherein the pump pipeline sections comprise a blood pump pipeline section configured to cooperate with one of the pump components so as to drive the blood to flow, the panel kit further comprises a blood drawing flow path that is configured to connect an input of the blood pump pipeline section to the patient's blood vessel and is provided with the blood drawing pressure monitoring pipeline section; and/or
   the pump pipeline sections comprise a blood pump pipeline section configured to cooperate with one of the pump components so as to drive the blood to flow, the panel kit further comprises a blood pumping flow path that is connected to an output of the blood pump pipeline section and a blood input of the first filter element respectively and is provided with the pressure monitoring pipeline section before filtering; and/or
   the pump pipeline sections comprise a waste liquid pressure pump pipeline section configured to cooperate with one of the pump components so as to drive the medical liquid to discharge from the first filter element, and the panel kit further comprises a waste liquid flow path that is connected to a medical liquid output of the first filter element and an input of the waste liquid pressure pump pipeline section respectively and is provided with the waste liquid pressure monitoring pipeline section; and/or
   the pump pipeline sections comprise a waste liquid pressure pump pipeline section configured to cooperate with one of the pump components so as to drive the medical liquid to discharge from the first filter element, the panel kit further comprises a waste liquid flow path and a collection flow path, the waste liquid flow path is connected to a medical liquid output of the first filter element and an input of the waste liquid pressure pump pipeline section respectively, an input of the collection flow path is connected to an output of the waste liquid pressure pump pipeline section, and the blood leakage monitoring pipeline section is arranged in the waste liquid flow path or the collection flow path.
20. The panel kit of any one of examples 1 to 4, wherein at least one of the monitoring pipeline sections comprises a pressure monitoring module comprising a fluid part, a gas part, a diaphragm and the second interface; a side cavity for fluid defined at least partially by the fluid part is separated from a side cavity for gas defined at least partially by the gas part through the diaphragm, the side cavity for fluid is used for the blood or the medical liquid to flow there-through, the second interface is fluidly communicated with the side cavity for gas, and if the second interface is connected to the first interface, the monitoring pipeline sections are used for the host to monitor a pressure parameter of the medical liquid or the blood flowing through the side cavity for fluid.
21. The panel kit of any one of examples 1 to 4, further comprising a heating component configured to be mounted on a heating region of the host, such that the medical liquid and/or the blood in the heating component is heated by a heating unit of the host.
22. A panel kit for a blood purification device, the blood purification device comprises a host provided with pump components, a conductive member and a first interface, and the panel kit comprises:
   a power component, comprising a power panel and at least two pump pipeline sections, wherein the power panel is configured to be detachably mounted to the host, the at least two pump pipeline sections are mounted to the power panel, at least one of the pump pipeline sections is configured to cooperate with at least a corresponding one of the pump components so as to drive a blood to flow along the at least one of the pump pipeline sections, and at least one of the pump pipeline sections is configured to cooperate with at least a corresponding one of the pump components so as to drive a medical liquid to flow along the at least one of the pump pipeline sections; if the power panel is mounted at a first target position of the host, the pump pipeline sections are adapted to the pump components respectively, and if the pump pipeline sections are adapted to the pump components respectively, the pump pipeline sections are capable of being driven by the pump components respectively to drive the corresponding blood or medical liquid to flow; and
   a monitoring component, comprising a monitoring panel, monitoring pipeline sections and an electrostatic discharge (ESD) element, wherein the monitoring panel is configured to be detachably mounted to the host, the monitoring panel is arranged separately from the power panel such that the monitoring panel and the power panel are independent of each other and can be assembled and disassembled separately, each of the monitoring pipeline sections and the ESD element are mounted to the monitoring panel, the monitoring pipeline sections are configured to monitor parameter information of the medical liquid and/or the blood, and the parameter information at least comprises a pressure parameter; at least one of the monitoring pipeline sections is provided with a second interface; the monitoring component is capable of being manually mounted such that the monitoring panel is mounted at a second target position of the host, the second interface is synchronously connected to the first interface and the ESD element is synchronously connected to the conductive member, and if the second interface is connected to the first interface, the monitoring pipeline sections are used for the host to monitor the parameter information, and if the ESD element is connected to the conductive member, the ESD element is capable of discharging static electricity from the panel kit.
23. A panel kit for a blood purification device, the blood purification device comprises a host provided with pump components, a conductive member and a retractable first interface, and the panel kit comprises:
   a power component, comprising a power panel and at least two pump pipeline sections, wherein the power panel is configured to be detachably mounted to the host, the at least two pump pipeline sections are mounted to the power panel, at least one of the pump pipeline sections is configured to cooperate with at least a corresponding one of the pump components so as to drive a blood to flow along the at least one of the pump pipeline sections, and at least one of the pump pipeline sections is configured to cooperate with at least a corresponding one of the pump components so as to drive a medical liquid to flow along the at least one of the pump pipeline sections; if the power panel is mounted at a first target position of the host, the pump pipeline sections are adapted to the pump components respectively, and if the pump pipeline sections are adapted to the pump components respectively, the pump pipeline sections are capable of being driven by the pump components respectively to drive the corresponding blood or medical liquid to flow; and
   a monitoring component, comprising a monitoring panel, monitoring pipeline sections and an electrostatic discharge (ESD) element, wherein the monitoring panel is configured to be detachably mounted to the host, the monitoring panel is arranged separately from the power panel such that the monitoring panel and the power panel are independent of each other and can be assembled and disassembled separately, each of the monitoring pipeline sections and the ESD element are mounted to the monitoring panel, the monitoring pipeline sections are configured to monitor parameter information of the medical liquid and/or the blood, and the parameter information at least comprises a pressure parameter; at least one of the monitoring pipeline sections is provided with a second interface; if the monitoring panel is mounted at a second target position of the host, the first interface can be extended to be connected to the second interface, the ESD element is connected to the conductive member fixedly arranged on the host, or the ESD element is connected to the conductive member extended toward an exterior of the host, and if the second interface is connected to the first interface, the monitoring pipeline sections are used for the host to monitor the parameter information, and if the ESD element is connected to the conductive member, the ESD element is capable of discharging static electricity from pipeline sections of the panel kit.
24. A panel kit for a blood purification device, the blood purification device comprises a host provided with pump components, a conductive member and a first interface, and the panel kit comprises:
   a power component, comprising a power panel and at least two pump pipeline sections, wherein the power panel is configured to be detachably mounted to the host, the at least two pump pipeline sections are mounted to the power panel, at least one of the pump pipeline sections is configured to cooperate with at least a corresponding one of the pump components so as to drive a blood to flow along the at least one of the pump pipeline sections, and at least one of the pump pipeline sections is configured to cooperate with at least a corresponding one of the pump components so as to drive a medical liquid to flow along the at least one of the pump pipeline sections; if the power panel is mounted at a first target position of the host, the pump pipeline sections are adapted to the pump components respectively, and if the pump pipeline sections are adapted to the pump components respectively, the pump pipeline sections are capable of being driven by the pump components respectively to drive the corresponding blood or medical liquid to flow; and
   a monitoring component, comprising a monitoring panel, monitoring pipeline sections and an electrostatic discharge (ESD) element, wherein the monitoring panel is configured to be detachably mounted to the host, the monitoring panel is arranged separately from the power panel such that the monitoring panel and the power panel are independent of each other and can be assembled and disassembled separately, each of the monitoring pipeline sections and the ESD element are mounted to the monitoring panel, the monitoring pipeline sections are configured to monitor parameter information of the medical liquid and/or the blood, and the parameter information at least comprises a pressure parameter; at least one of the monitoring pipeline sections is provided with a second interface; if the monitoring panel is mounted at a second preset position of the host, the host is capable of driving the monitoring panel to move to a second target position, such that the second interface is connected to the first interface and the ESD element is connected to the conductive member, and if the second interface is connected to the first interface, the monitoring pipeline sections are used for the host to monitor the parameter information, and if the ESD element is connected to the conductive member, the ESD element is capable of discharging static electricity from pipeline sections of the panel kit.
25. A panel kit for a blood purification device, the blood purification device comprises a host provided with pump components, a conductive member and a first interface, and the panel kit comprises:
   a power component, comprising a power panel and at least two pump pipeline sections, wherein the power panel is configured to be detachably mounted to the host, the at least two pump pipeline sections are mounted to the power panel, at least one of the pump pipeline sections is configured to cooperate with at least a corresponding one of the pump components so as to drive a blood to flow along the at least one of the pump pipeline sections, and at least one of the pump pipeline sections is configured to cooperate with at least corresponding one of the pump components so as to drive a medical liquid to flow along the at least one of the pump pipeline sections; if the power panel is mounted at a first target position of the host, the pump pipeline sections are adapted to the pump components respectively, and if the pump pipeline sections are adapted to the pump components respectively, the pump pipeline sections are capable of being driven by the pump components respectively to drive the corresponding blood or medical liquid to flow; and
   a monitoring component, comprising a monitoring panel, monitoring pipeline sections and an electrostatic discharge (ESD) element, wherein the monitoring panel is configured to be detachably mounted to the host, the monitoring panel is arranged separately from the power panel such that the monitoring panel and the power panel are independent of each other and can be assembled and disassembled separately, each of the monitoring pipeline sections and the ESD element are mounted to the monitoring panel, the monitoring pipeline sections are configured to monitor parameter information of the medical liquid and/or the blood, and the parameter information at least comprises a pressure parameter; at least one of the monitoring pipeline sections is provided with a second interface; the second interface is movably arranged relative to the monitoring panel, and if the monitoring panel is mounted at a second target position of the host, the second interface is capable of being manually operated to achieve connection of the first interface to the second interface, and if the second interface is connected to the first interface, the monitoring pipeline sections are used for the host to monitor the parameter information, and if the ESD element is connected to the conductive member, the ESD element is capable of discharging static electricity from the panel kit.
26. The panel kit of any one of examples 22 to 25, wherein the pump pipeline sections comprise a first flexible pipeline section abutting against a pump head of one of the pump components if the pump pipeline sections are adapted to the pump components respectively, such that rotation of the pump head is capable of driving the first flexible pipeline to be wound around the pump head.
27. The panel kit of any one of examples 22 to 25, further comprising a second flexible pipeline section, wherein the monitoring pipeline sections comprise at least one monitoring pipeline section, the second flexible pipeline section is connected between at least one of the pump pipeline sections and at least one of the monitoring pipeline sections.
28. The panel kit of any one of examples 22 to 25, wherein the monitoring pipeline sections comprise at least one monitoring pipeline section, and at least one of the monitoring pipeline sections comprises a pressure monitoring module comprising a fluid part, a gas part, a diaphragm and the second interface; a side cavity for fluid defined at least partially by the fluid part is separated from a side cavity for gas defined at least partially by the gas part through the diaphragm, the side cavity for fluid is used for the blood or the medical liquid to flow there-through, the second interface is fluidly communicated with the side cavity for gas, and if the second interface is connected to the first interface, the monitoring pipeline sections are used for the host to monitor a pressure parameter of the medical liquid or the blood flowing through the side cavity for fluid.
29. The panel kit of any one of examples 22 to 25, further comprising a heating component configured to be mounted on a heating region of the host, such that the medical liquid and/or the blood in the heating component is heated by a heating unit of the host.

## Claims

1. A panel kit (100) for a blood purification device, **characterized in that**, the blood purification device comprises a host (200) provided with pump components (21) and a first interface (22), and the panel kit (100) comprises:
a power component (11), comprising a power panel (111) and at least two pump pipeline sections (112), wherein the power panel (111) is configured to be detachably mounted to the host (200), the at least two pump pipeline sections (112) are mounted to the power panel (111), at least one of the pump pipeline sections (112) is configured to cooperate with at least a corresponding one of the pump components (21) so as to drive a blood to flow along the at least one of the pump pipeline sections (112), and at least one of the pump pipeline sections (112) is configured to cooperate with at least another one of the pump components (21) so as to drive a medical liquid to flow along the at least one of the pump pipeline sections (112); if the power panel (111) is mounted at a first target position of the host (200), the pump pipeline sections (112) are adapted to the pump components (21) respectively, and if the pump pipeline sections (112) are adapted to the pump components (21) respectively, the pump pipeline sections (112) are capable of being driven by the pump components (21) respectively to drive the corresponding blood or medical liquid to flow; and
a monitoring component (12), comprising a monitoring panel (121) and at least two monitoring pipeline sections (122), wherein the monitoring panel (121) is configured to be detachably mounted to the host (200), the monitoring panel (121) is arranged separately from the power panel (111) such that the monitoring panel (121) and the power panel (111) are independent of each other and can be assembled and disassembled separately, the at least two monitoring pipeline sections (122) are mounted to the monitoring panel (121) and are configured to monitor parameter information of the medical liquid and/or the blood, and the parameter information at least comprises a pressure parameter; at least one of the monitoring pipeline sections (122) is provided with a second interface (1221); the monitoring component (12) is capable of being manually mounted such that the monitoring panel (121) is mounted at a second target position of the host (200) and the second interface (1221) is synchronously connected to the first interface (22), and if the second interface (1221) is connected to the first interface (22), the monitoring pipeline sections (122) are used for the host (200) to monitor the parameter information.

2. A panel kit (100) for a blood purification device, **characterized in that**, the blood purification device comprises a host (200) provided with pump components (21) and a retractable first interface (22), and the panel kit (100) comprises:
a power component (11), comprising a power panel (111) and at least two pump pipeline sections (112), wherein the power panel (111) is configured to be detachably mounted to the host (200), the at least two pump pipeline sections (112) are mounted to the power panel (111), at least one of the pump pipeline sections (112) is configured to cooperate with at least a corresponding one of the pump components (21) so as to drive a blood to flow along the at least one of the pump pipeline sections (112), and at least one of the pump pipeline sections (112) is configured to cooperate with at least corresponding one of the pump components (21) so as to drive a medical liquid to flow along the at least one of the pump pipeline sections (112); if the power panel (111) is mounted at a first target position of the host (200), the pump pipeline sections (112) are adapted to the pump components (21) respectively, and if the pump pipeline sections (112) are adapted to the pump components (21) respectively, the pump pipeline sections (112) are capable of being driven by the pump components (21) respectively to drive the corresponding blood or medical liquid to flow; and
a monitoring component (12), comprising a monitoring panel (121) and at least two monitoring pipeline sections (122), wherein the monitoring panel (121) is configured to be detachably mounted to the host (200), the monitoring panel (121) is arranged separately from the power panel (111) such that the monitoring panel (121) and the power panel (111) are independent of each other and can be assembled and disassembled separately, the at least two monitoring pipeline sections (122) are mounted to the monitoring panel (121) and are configured to monitor parameter information of the medical liquid and/or the blood, and the parameter information at least comprises a pressure parameter; at least one of the monitoring pipeline sections (122) is provided with a second interface (1221), and if the monitoring panel (121) is mounted at a second target position of the host (200), the first interface (22) can be extended to be connected to the second interface (1221), and if the second interface (1221) is connected to the first interface (22), the monitoring pipeline sections (122) are used for the host (200) to monitor the parameter information.

3. A panel kit (100) for a blood purification device, **characterized in that**, the blood purification device comprises a host (200) provided with pump components (21) and a first interface (22), and the panel kit (100) comprises:
a power component (11), comprising a power panel (111) and at least two pump pipeline sections (112), wherein the power panel (111) is configured to be detachably mounted to the host (200), the at least two pump pipeline sections (112) are mounted to the power panel (111), at least one of the pump pipeline sections (112) is configured to cooperate with at least a corresponding one of the pump components (21) so as to drive a blood to flow along the at least one of the pump pipeline sections (112), and at least one of the pump pipeline sections (112) is configured to cooperate with at least corresponding one of the pump components (21) so as to drive a medical liquid to flow along the at least one of the pump pipeline sections (112); if the power panel (111) is mounted at a first target position of the host (200), the pump pipeline sections (112) are adapted to the pump components (21) respectively, and if the pump pipeline sections (112) are adapted to the pump components (21) respectively, the pump pipeline sections (112) are capable of being driven by the pump components (21) respectively to drive the corresponding blood or medical liquid to flow; and
a monitoring component (12), comprising a monitoring panel (121) and at least two monitoring pipeline sections (122), wherein the monitoring panel (121) is configured to be detachably mounted to the host (200), the monitoring panel (121) is arranged separately from the power panel (111) such that the monitoring panel (121) and the power panel (111) are independent of each other and can be assembled and disassembled separately, the at least two monitoring pipeline sections (122) are mounted to the monitoring panel (121) and are configured to monitor parameter information of the medical liquid and/or the blood, and the parameter information at least comprises a pressure parameter; at least one of the monitoring pipeline sections (122) is provided with a second interface (1221); if the monitoring panel (121) is mounted at a second preset position of the host (200), the host (200) is capable of driving the monitoring panel (121) to move to a second target position, such that the second interface (1221) is connected to the first interface (22), and if the second interface (1221) is connected to the first interface (22), the monitoring pipeline sections (122) are used for the host (200) to monitor the parameter information.

4. A panel kit (100) for a blood purification device, **characterized in that**, the blood purification device comprises a host (200) provided with pump components (21) and a first interface (22), and the panel kit (100) comprises:
a power component (11), comprising a power panel (111) and at least two pump pipeline sections (112), wherein the power panel (111) is configured to be detachably mounted to the host (200), the at least two pump pipeline sections (112) are mounted to the power panel (111), at least one of the pump pipeline sections (112) is configured to cooperate with at least a corresponding one of the pump components (21) so as to drive a blood to flow along the at least one of the pump pipeline sections (112), and at least one of the pump pipeline sections (112) is configured to cooperate with at least corresponding one of the pump components (21) so as to drive a medical liquid to flow along the at least one of the pump pipeline sections (112); if the power panel (111) is mounted at a first target position of the host (200), the pump pipeline sections (112) are adapted to the pump components (21) respectively, and if the pump pipeline sections (112) are adapted to the pump components (21) respectively, the pump pipeline sections (112) are capable of being driven by the pump components (21) respectively to drive the corresponding blood or medical liquid to flow; and
a monitoring component (12), comprising a monitoring panel (121) and at least two monitoring pipeline sections (122), wherein the monitoring panel (121) is configured to be detachably mounted to the host (200), the monitoring panel (121) is arranged separately from the power panel (111) such that the monitoring panel (121) and the power panel (111) are independent of each other and can be assembled and disassembled separately, the at least two monitoring pipeline sections (122) are mounted to the monitoring panel (121) and are configured to monitor parameter information of the medical liquid and/or the blood, and the parameter information at least comprises a pressure parameter; at least one of the monitoring pipeline sections (122) is provided with a second interface (1221); the second interface (1221) is movably arranged relative to the monitoring panel (121), and if the monitoring panel (121) is mounted at a second target position of the host (200), the second interface (1221) is capable of being manually operated to achieve connection of the first interface (22) to the second interface (1221), and if the second interface (1221) is connected to the first interface (22), the monitoring pipeline sections (122) are used for the host (200) to monitor the parameter information.

5. The panel kit (100) of any one of claims 1 to 3, wherein the second interface (1221) is fixedly arranged relative to the monitoring panel (121); or
the second interface (1221) is movably arranged relative to the monitoring panel (121).

6. The panel kit (100) of any one of claims 1 to 4, wherein the pump pipeline sections (112) comprise a first flexible pipeline section abutting against a pump head of one of the pump components (21) if the pump pipeline sections (112) are adapted to the pump components (21) respectively, such that rotation of the pump head is capable of driving the first flexible pipeline to be wound around the pump head.

7. The panel kit (100) of any one of claims 1, 2 and 4, wherein whether the monitoring panel (121) is mounted at the second target position, is detectable by a second position detection mechanism (26) on the host (200).

8. The panel kit (100) of claim 3, wherein if the monitoring panel (121) is mounted at the second preset position, the second interface (1221) is not connected to the first interface (22); and/or
whether the monitoring panel (121) is mounted at the second preset position and/or the second target position, is detectable by a second position detection mechanism (26) on the host (200); and/or
the monitoring panel (121) is provided with a second connection part (1211) configured to be connectable to a second driving mechanism (25) of the host (200), such that the second driving mechanism (25) is capable of driving the monitoring panel (121) to move from the second preset position to the second target position.

9. The panel kit (100) of any one of claims 1 to 4, further comprising a second flexible pipeline section connected between at least one of the pump pipeline sections (112) and at least one of the monitoring pipeline sections. (122).

10. The panel kit (100) of any one of claims 1 to 4, wherein at least two of the monitoring pipeline sections (122) are provided with the second interfaces (1221) respectively, and the first interface (22) comprises at least two first interfaces (22), and the second interfaces (1221) are configured to be connected to the corresponding first interfaces (22) on the host (200) respectively, such that the host (200) is capable of monitoring the pressure parameter of the corresponding blood or medical liquid through the monitoring pipeline sections (122); and/or
the blood purification device further comprises a first filter element (300) configured to filter the blood flowing through the pump pipeline sections (112); if the second interfaces (1221) are connected to the first interfaces (22) respectively, the monitoring pipeline sections (122) are used for the host (200) to monitor the pressure parameter of the corresponding blood or medical liquid; the at least two monitoring pipeline sections (122) further comprise a blood leakage monitoring pipeline section (122d) configured to cooperate with a blood leakage monitoring module on the host (200), so as to monitor whether the blood at one side of a filtration membrane of the first filter element (300) permeates to the other side of the filtration membrane.

11. The panel kit (100) of any one of claims 1 to 4, wherein the blood purification device further comprises a first filter element (300) configured to filter the blood flowing through the pump pipeline sections (112);
the monitoring pipeline sections (122) comprise at least one of a blood leakage monitoring pipeline section (122d), a blood drawing pressure monitoring pipeline section (122a), a pressure monitoring pipeline section before filtering (122b) and a waste liquid pressure monitoring pipeline section (122c);
the blood leakage monitoring pipeline section (122d) is configured to cooperate with a blood leakage monitoring module on the host (200), so as to monitor whether the blood at one side of a filtration membrane of the first filter element (300) permeates to the other side of the filtration membrane;
the blood drawing pressure monitoring pipeline section (122a) is configured to monitor the pressure parameter of the blood drawn out of a patient's blood vessel;
the pressure monitoring pipeline section before filtering (122b) is configured to monitor a pressure parameter of the blood flowing from the pump pipeline sections (112) into the first filter element (300);
the waste liquid pressure monitoring pipeline section (122c) is configured to monitor a pressure parameter of the medical liquid discharged from the first filter element (300).

12. The panel kit (100) of claim 11, wherein the blood purification device further comprises a second filter element (400) configured to re-filter the blood flowing through the pump pipeline sections (112), a blood input of the second filter element (400) is fluidly communicated with a blood output of the first filter element (300) so as to re-filter the blood flowing out of the blood output of the first filter element (300);
the monitoring pipeline sections (122) further comprise a secondary membrane pressure monitoring pipeline section (122e) configured to fluidly communicate the first filter element (300) with the second filter element (400) and to monitor the pressure parameter of the blood.

13. The panel kit (100) of claim 12, wherein the monitoring component (12) comprises at least two monitoring components (12), any two of the monitoring components (12) are arranged separately from each other, such that each of the monitoring components (12) can be assembled and disassembled separately, and each of the monitoring components (12) is provided with at least two of the blood leakage monitoring pipeline section (122d), the blood drawing pressure monitoring pipeline section (122a), the pressure monitoring pipeline section before filtering (122b) and the waste liquid pressure monitoring pipeline section (122c).

14. The panel kit (100) of claim 11, wherein the pump pipeline sections (112) comprise a blood pump pipeline section (1121) configured to cooperate with one of the pump components (21) so as to drive the blood to flow, the panel kit (100) further comprises a blood drawing flow path (1301) that is configured to connect an input of the blood pump pipeline section (1121) to the patient's blood vessel and is provided with the blood drawing pressure monitoring pipeline section (122a); and/or
the pump pipeline sections (112) comprise a blood pump pipeline section (1121) configured to cooperate with one of the pump components (21) so as to drive the blood to flow, the panel kit (100) further comprises a blood pumping flow path (1302) that is connected to an output of the blood pump pipeline section (1121) and a blood input of the first filter element (300) respectively and is provided with the pressure monitoring pipeline section before filtering (122b); and/or the pump pipeline sections (112) comprise a waste liquid pressure pump pipeline section (1122) configured to cooperate with one of the pump components (21) so as to drive the medical liquid to discharge from the first filter element (300), and the panel kit (100) further comprises a waste liquid flow path (1303) that is connected to a medical liquid output of the first filter element (300) and an input of the waste liquid pressure pump pipeline section (1122) respectively and is provided with the waste liquid pressure monitoring pipeline section (122c); and/or
the pump pipeline sections (112) comprise a waste liquid pressure pump pipeline section (1122) configured to cooperate with one of the pump components (21) so as to drive the medical liquid to discharge from the first filter element (300), the panel kit (100) further comprises a waste liquid flow path (1303) and a collection flow path (1315), the waste liquid flow path (1303) is connected to a medical liquid output of the first filter element (300) and an input of the waste liquid pressure pump pipeline section (1122) respectively, an input of the collection flow path (1315) is connected to an output of the waste liquid pressure pump pipeline section (1122), and the blood leakage monitoring pipeline section (122d) is arranged in the waste liquid flow path (1303) or the collection flow path (1315).

15. The panel kit of any one of claims 1 to 4, further comprising a heating component (15) configured to be mounted on a heating region of the host (200), such that the medical liquid and/or the blood in the heating component (15) is heated by a heating unit of the host (200).
